# EUROPEAN PATENT SPECIFICATION

(11) **EP 0 549 107 B1**
(45) Date of publication and mention of the grant of the patent: **29.12.1997**
(21) Application number: 92309231.6
(22) Date of filing: 09.10.1992
(51) Int. Cl.: C12Q 1/68, C12P 19/34

(54) **Method for producing a polynucleotide for use in single primer amplification and phosphorothioate-containing oligonucleotides as primers in nucleic acid amplification**
Verfahren zur Herstellung eines Polynukleotids zum Gebrauch in "single-primer" Amplifizierung und Phosphorothioat-enthaltende Oligonukleotide als Primer in Nukleinsäureamplifizierung
Méthode pour produire un polynucléotide qui peut être utilisé pour des amplifications à amorce unique et utilisation d'oligonucléotides contenant le phosphorotioate comme amorces pour l'amplification d'acides nucléiques

(30) Priority: 11.10.1991 US 776538; 31.07.1992 US 923070
(43) Date of publication of application: 30.06.1993
(73) Proprietor: BEHRINGWERKE Aktiengesellschaft, 35001 Marburg (DE)
(72) Inventor: Western, Linda M., San Mateo, CA 94401 (US); Hahnenberger, Karen M., Cupertino, Ca 95014 (US); Rose, Samuel, Mountain View, CA 94040 (US); Becker, Martin, Palo Alto, CA 94303 (US); Ullman, Edwin F., Atherton, CA 94025 (US); McGall, Glenn H., Mountain View, CA 94040 (US)
(74) Representative: Armitage, Ian Michael

(56) References cited:
- EP-A- 0 379 369
- EP-A- 0 469 755
- WO-A-86/07612
- NUCLEIC ACIDS RESEARCH. vol. 19, no. 11, 11 June 1991, ARLINGTON, VIRGINIA US pages 3139 - 3141 H. LI ET AL. 'Eliminating primers from completed polymerase chain reactions with exonuclease VII'
- NUCLEIC ACIDS RESEARCH. vol. 20, no. 14, 25 July 1992, ARLINGTON, VIRGINIA US pages 3551 - 3554 A. SKERRA 'Phosphorothioate primers improve the amplification of DNA sequences by DNA polymerases with proofreading activity'

## Description

### BACKGROUND OF THE INVENTION

### 1. Field of the Invention.

Nucleic acid hybridization has been employed for investigating the identity and establishing the presence of nucleic acids. Hybridization is based on complementary base pairing. When complementary single stranded nucleic acids are incubated together, the complementary base sequences pair to form double stranded hybrid molecules. The ability of single stranded deoxyribonucleic acid (ssDNA) or ribonucleic acid (RNA) to form a hydrogen bonded structure with a complementary nucleic acid sequence has been employed as an analytical tool in molecular biology research. The availability of radioactive nucleoside triphosphates of high specific activity and the ³²P labelling of DNA with T4 kinase has made it possible to identify, isolate, and characterize various nucleic acid sequences of biological interest. Nucleic acid hybridization has great potential in diagnosing disease states associated with unique nucleic acid sequences. These unique nucleic acid sequences may result from genetic or environmental change in DNA by insertions, deletions, point mutations, or by acquiring foreign DNA or RNA by means of infection by bacteria, molds, fungi, and viruses. Nucleic acid hybridization has, until now, been employed primarily in academic and industrial molecular biology laboratories. The application of nucleic acid hybridization as a diagnostic tool in clinical medicine is limited because of the frequently very low concentrations of disease related DNA or RNA present in a patient's body fluid and the unavailability of a sufficiently sensitive method of nucleic acid hybridization analysis.

Current methods for detecting specific nucleic acid sequences generally involve immobilization of the target nucleic acid on a solid support such as nitrocellulose paper, cellulose paper, diazotized paper, or a nylon membrane. After the target nucleic acid is fixed on the support, the support is contacted with a suitably labelled probe nucleic acid for about two to forty-eight hours. After the above time period, the solid support is washed several times at a controlled temperature to remove unhybridized probe. The support is then dried and the hybridized material is detected by autoradiography or by spectrometric methods.

When very low concentrations must be detected, the current methods are slow and labor intensive, and nonisotopic labels that are less readily detected than radiolabels are frequently not suitable. A method for increasing the sensitivity to permit the use of simple, rapid, nonisotopic, homogeneous or heterogeneous methods for detecting nucleic acid sequences is therefore desirable.

Recently, a method for the enzymatic amplification of specific segments of DNA known as the polymerase chain reaction (PCR) method has been described. This in vitro amplification procedure is based on repeated cycles of denaturation, oligonucleotide primer annealing, and primer extension by thermophilic polymerase, resulting in the exponential increase in copies of the region flanked by the primers. The PCR primers, which anneal to opposite strands of the DNA, are positioned so that the polymerase catalyzed extension product of one primer can serve as a template strand for the other, leading to the accumulation of a discrete fragment whose length is defined by the distance between the 5' ends of the oligonucleotide primers.

Another method has also recently been described for amplifying nucleic acid sequences. This method is referred to as single primer amplification because, unlike PCR, it utilizes only one primer, not two as required by PCR. This method provides for the amplification of a target sequence that possesses a stem-loop or inverted repeat structure where the target sequence is flanked by relatively short complementary sequences. Various methods for creating such a target sequence in relation to the presence of a polynucleotide analyte to be detected have also been described.

One problem with the use of synthetic oligonucleotide primers is that such primers are susceptible to degradation by the exonuclease activities associated with many DNA polymerases used in amplification procedures. One such procedure, as mentioned above, is PCR, which provides for an exponential amplification of nucleic acids. PCR is generally carried out using a Taq polymerase, which lacks a 3'-5' exonuclease activity and is not capable of removing mismatches. Numerous modifications of this procedure have been described, some requiring temperature cycling, others amplifying at a single temperature. Those maintaining a single temperature rely on the activities of as many as four enzymes to achieve amplification, whereas procedures involving temperature cycling depend on heat-stable polymerases. The detection of genetic polymorphisms or point mutations in diagnosing genetic disease is routinely described using PCR. Primers mismatched at the 3' end are used to introduce artificial restriction sites or to detect single base mismatches in the target DNA. Enzymes containing 3' exonuclease activities used in amplification protocols, however, would digest the mismatched primer-template region, severely limiting this type of analysis.

One part of the invention described herein permits extension of primers along templates and amplification of primers even in the presence of enzymes containing strong 3' exonuclease activities.

### 2. Description of the Prior Art.

A process for amplifying, detecting and/or cloning nucleic acid sequences is disclosed in U.S. Patent Nos. 4,683,195 and 4,683,202. Sequence polymerization by polymerase chain reaction is described by Saiki, et al., (1986) Science, 230: 1350-1354. A method of making an oligonucleotide is described in European Patent Application No. 0194545 A2. Belgian Patent Application No. BE 904402 discloses a mold for making DNA detection probes. Gene amplification in eukaryotic cells is disclosed in U.S. Patent No. 4,656,134.

Langer, et al., Proc. Natl. Acad. Sci. USA, (1981) 78: 6633-6637 discloses the enzymatic synthesis of biotin labelled polynucleotides and the use of these materials as novel nucleic acid affinity probes. The detection of viral genomes in cultured cells and paraffin imbedded tissue sections using biotin labelled hybridization probes is discussed by Brigati, et al., Virology, (1983) 126: 32-50. U.S. Patent No. 4,486,539 discloses the detection of microbial nucleic acids by a one step sandwich hybridization test. Sensitive tests for malignancies based on DNA detection is described in U.S. Patent No. 4,490,472. U.S. Patent No. 4,480,040 discloses the sensitive and rapid diagnosis of plant viroid diseases and viruses employing radioactively labelled DNA that is complementary to the viroid or to the nucleic acid of the virus being diagnosed. European Patent Application 83106112.2 (EP-A-97373) (Priority U.S. Patent Application 391,440 filed June 23, 1982) teaches modified labelled nucleotides and polynucleotides and methods of preparing, utilizing, and detecting the same. Methods and compositions for the detection and determination of cellular DNA are disclosed in U.S. Patent No. 4,423,153. Specific DNA probes in diagnostic microbiology are discussed in U.S. Patent No. 4,358,535. A method for detection of polymorphic restriction sites and nucleic acid sequences is discussed in European Patent Application No. 0164054 A1. U.S. Patent No. 4,663,283 describes a method of altering double-stranded DNA.

Genomic amplification with transcript sequencing is discussed by Stoflet, et al., Science (198) 239:491. Primer-directed enzymatic amplification of DNA with a thermostable DNA polymerase is described by Saiki, et al., Science (1988) 239:487. U.S. Patent No. 4,724,202 discloses the use of non-hybridizable nucleic acids for the detection of nucleic acid hybridization. Bugawan, et al., describe the use of non-radioactive oligonucleotide probes to analyze enzymatically amplified DNA for prenatal diagnosis and forensic HLA typing.

Detection and isolation of homologous, repeated and amplified nucleic acid sequences is disclosed in U.S. Patent No. 4,675,283. U.S. Patent Nos. 4,683,195 and 4,683,202 disclose a homogeneous polynucleotide displacement assay with digestion of the displaced RNA single strand polynucleotide from the reagent complex and amplifying nucleic acid sequences with treatment of separate complementary strands of the nucleic acid with two oligonucleotide primers. European Patent Application No. 0200362 describes a process for amplifying, detecting or cloning nucleic acid sequences and useful in disease diagnosis and in preparation of transformation vectors. A method for simple analysis of relative nucleic acid levels in multiple small samples by cytoplasmic dot hybridization is described in U.S. Patent No. 4,677,054. A hybridization method of detecting nucleic acid sequences with a probe containing a thionucleotide is described in U.S. Patent No. 4,647,529.

A simple and efficient enzymatic method for covalent attachment of DNA to cellulose and its application for hybridization-restriction analysis and for in vitro synthesis of DNA probes is described in Nucleic Acids Research (1986) 14: 9171-9191. Cleavage of single stranded oligonucleotides by Eco RI restriction endonuclease is described in Nucleic Acid Research (1987) 15: 709-716.

Exponential Amplification of Recombinant-RNA Hybridization Probes is described by Lizardi, et al. (1988) Bio/Technology 6:1197-1202. Fahrlander, et al., discusses Amplifying DNA Probe Signals: A Christmas Tree Approach in Bio/Technology (1988) 6:1165-1168.

A nucleic acid hybridization assay employing probes cross-linkable to target sequences is described in U.S. Patent No. 4,599,303. The method involves the preparation of a specific single stranded ribonucleic acid or deoxyribonucleic acid molecule into which a bifunctional cross-linking molecule has been covalently incorporated. The incorporation is such that the cross-linking molecule retains the capacity to undergo a second reaction with the nucleic acid of the bacterial, viral, or mammalian chromosome, which is the target for the probe such as to form a covalent cross link. Following cross-linking, the uncrossed link probe is separated from covalently cross-linked probe-target complex using one of several procedures which differentiate between single stranded probe and double stranded covalently linked probe-target complex.

A hybridization method and probe for detecting nucleic acid sequences is described in U.S. Patent No. 4,908,307. An amplified hybridization assay is described in U.S. Patent No. 4,882,269 wherein a family of signal-generating secondary probes bind to a primary probe that hybridizes to the target sequence of interest.

Detection of target sequences in nucleic acids by hybridization using diagnostic and contiguous probes for diagnosis of genetic abnormality diseases, especially in an automated procedure, is described in European Patent Application No. 0 185 494A2. In the method a sample is hybridized with a probe complementary to a diagnostic portion of the target sequence (the diagnostic probe) and with a probe complementary to a nucleotide sequence contiguous with the diagnostic portion (the contiguous probe) under conditions wherein the diagnostic probe remains bound substantially only to the sample nucleic acid containing the target sequence. The diagnostic probe and contiguous probe are then covalently attached to yield a target probe that is complementary to the target sequence and the probes which are not attached are removed. In a preferred mode, one of the probes is labeled so that the presence or absence of the target sequence can then be tested by melting the sample nucleic acid target probe duplex, eluting the dissociated target probe, and testing for the label.

The above method suffers at least one disadvantage in that contiguous sequences are required. To carry out the method, one must identify the diagnostic sequence and the contiguous sequence and create diagnostic and contiguous probes complementary to the above sequences. If the diagnostic and contiguous sequences are not identified precisely, then the diagnostic and contiguous probes may not hybridize sufficiently and the assay specificity and sensitivity can be lost or substantially decreased.

A DNA amplification and subtraction technique is described in W089/12695. The method involves isolating genomic or RNA-derived duplex fragments which are unique to one of two fragment mixtures. The fragments in positive-source and negative-source mixtures are separately equipped with end linkers, and each mixture is amplified by successive primed-strand replications, using a single primer which is homologous to the associated linker. The second source linker is biotinylated, and the fragments in this mixture are hybridized in molar excess with the fragments in the positive source mixture. DNA species which are not hybridized with the biotinylated species, i.e., species that are unique to the positive source mixture, are isolated after removal of hybridized species by affinity chromatography. Also disclosed is a method of amplifying a mixture of DNA fragments by repeated linker/primer replication.

U.S. Patent Applications Serial Nos. 07/299,282 and 07/399,795, filed January 19, 1989, and August 29, 1989, respectively, see also EP379369, describe nucleic acid amplification using a single polynucleotide primer. U.S. Patent Applications Serial No. 07/555,323 filed July 19, 1990, see also EP469755, discloses methods for producing a polynucleotide for use in single primer amplification. The disclosures of these applications are incorporated herein by reference.

DNA and RNA sequence determination based on phosphorothioate chemistry is described by G. Gish, et al., in Science (1988) 240:1520-1522. A hybridization method of detecting nucleic acid sequences with a probe containing a thionucleotide is described in U.S. Patent No. 4,647,529. PCR-based site-directed mutagenesis using primers with mismatched 3'-ends is discussed by M. Nassal, et al., in Nucleic Acids Research (1990) 18(10):3077-3078. G. Sarkar, et al., disclose the characterization of polymerase chain reaction amplification of specific alleles in Analytical Biochemistry (1990) 186:64-68. Allele-specific enzymatic amplification of B-globin genomic DNA for diagnosis of sickle cell anemia is described by D. Wu, et al., in Proc. Natl. Acad. Sci. USA (1989) 86:2757-2760. Modification of enzymatically amplified DNA for the detection of point mutations is disclosed by A. Haliassos, et al., in Nucleic Acids Research (1989) 17:3606. S. Kwok et al., describe the effects of primer-template mismatches on the polymerase chain reaction: human immunodeficiency virus type 1 model studies in Nucleic Acids Research (1990) :999-1005. The technical aspects of typing for HLA-DP alleles using allele-specific DNA in vitro amplification and sequence-specific oligonucleotide probes and the detection of single base mismatches is discussed by L. Fugger, et al., J. al., Immunol. Methods (1990) 129:175-185. J. Ott, et describe the protection of oligonucleotide primers against degradation by DNA polymerase I in Biochemistry (1987) 26:8237-8241. The duplex stability of phosphorothioate, methylphosphonate, and RNA analogs of two DNA 14-mers is disclosed by Kibler-Herzog, et al., Nucleic Acids Research (1991) 19(No.11):2979-2981. The effect of phosphorothioate homo-oligodeoxynucleotides on herpes simplex virus type 2-induced DNA polymerase is discussed by Gao, et al., J. Biol. Chem. (1989) 262 (No. 19):11521-11526, who observed inhibition of HSV-2 induced DNA polynerase and some human DNA polymerases by sulfur-containing oligonucleotides. Griep, et al., Biochemistry (1990) 29:9006-9014 disclose the reduction of the potent DNA polymerase III holoenzyme 3' to 5' exonuclease activity by template-primer analogues.

The use of tetraethylthiuram disulfide (TETD) as a reagent for phosphorothioate oligonucleotide synthesis via phosphoramidite chemistry is discussed in several promotional publications by Applied Biosystems, namely, 1) Research News, "DNA Synthesis Tetraethylthiuram Disulfide: A New Reagent for Antisense Phosphorothioate DNA," 2) User Bulletin Number 58, February 1991, Model 380A, 380B, 381A, 391, 392, 394 DNA Synthesizers and 3) promotional note for TETD/Acetonitrile, Part No. 401147, "TETD:Antisense Phosphorothioate Synthesis Made Easy."

A process for amplifying, detecting and/or cloning nucleic acid sequences is disclosed in U.S. Patent Nos. 4,683,195, 4,683,202, 4,800,159, 4,965,188 and 5,008,182. Sequence polymerization by polymerase chain reaction is described by Saiki, et al., (1986) Science, 230: 1350-1354. Primer-directed enzymatic amplification of DNA with a thermostable DNA polymerase is described by Saiki, et al., Science (1988) 239:487.

Amplification of nucleic acid sequences using oligonucleotides of random sequence as primers is described in U.S. Patent No. 5,043,272. The use of phosphorothioate primers in PCR amplification is described in just published Nucleic Acid Research, Vol 20, No. 14, 3551-3554, 1992.

### SUMMARY OF THE INVENTION

In one embodiment of the present invention a method is described for forming from an extender probe and a single stranded target polynucleotide sequence a single stranded polynucleotide sequence, which is free of unmodified extender probe, having a sequence identical to the target polynucleotide sequence attached at its 3'-end to a polynucleotide sequence complementary to a polynucleotide sequence at the 5'-end of the single stranded target polynucleotide sequence. The method comprises: (a) hybridizing to the 3'-end of the single stranded target polynucleotide sequence the 3'-end of the extender probe wherein the extender probe contains a sequence substantially identical to a sequence S2 at the 5'-end of the target polynucleotide sequence, (b) extending the extender probe along the single stranded target polynucleotide sequence, and (c) modifying the 3'-end of the extender probe not hybridized to the single stranded target polynucleotide sequence, (d) hybridizing a primer to the 3'-end of the extended extender probe, the primer having sequence S2 at its 3'-end and (e) extending the primer along the extended extender probe.

The invention disclosed herein includes methods and reagents for extending an extender probe to form a single stranded polydeoxynucleotide having two segments that are non-contiguous and complementary with each other wherein extender probe not involved in such extension is modified at its 3'-end. The method finds particular application, for example, in single primer amplification assays.

In one embodiment of the invention an extender probe is extended to produce a single stranded polydeoxynucleotide having two segments that are non-contiguous and complementary with each other. The method of production comprises the steps of (1) providing in combination (a) a polynucleotide having two non-contiguous, non-complementary nucleotide sequences, S1 and S2, wherein S2 is 5' of S1 and is at least ten nucleotides long, (b) an extender probe comprised of two deoxynucleotide sequences, wherein the sequence at the 3' end of the extender probe (EP1) is hybridizable with S1 and the other of the deoxynucleotide sequences (EP2) is substantially identical to S2 and (c) means for modifying the 3'-end of the extender probe that does not hybridize with the polynucleotide and (d) extending the extender probe along the polynucleotide wherein extender probe not hybridized to the polynucleotide becomes modified at its 3'-end.

In the above embodiment of the present invention a polydeoxynucleotide primer capable of hybridizing at least at its 3'-end with a nucleotide sequence complementary to S2, DNA polymerase, and deoxynucleoside triphosphates are provided in the combination under conditions where (a) the extender probe is extended along the polynucleotide to form a duplex, (b) the 3'-end of the extender probe not hybridized with the polynucleotide is modified, (c) the extended extender probe is dissociated from the duplex, (d) the polydeoxynucleotide primer hybridizes with and is extended along the extended extender probe to form a second duplex comprising extended primer, (e) the extended primer is dissociated from the second duplex, and (f) the primer hybridizes with and is extended along the extended primer to form a duplex comprising extended primer, and steps (e) and (f) are repeated.

Another embodiment of the invention is a method for forming a single stranded polynucleotide sequence complementary to a single stranded target polynucleotide sequence. The method comprises: (a) combining in a medium the single stranded target polynucleotide sequence, a DNA polymerase with 3' exonuclease activity, and an extender probe comprised of a sequence complementary to a sequence at the 3'-end of the single stranded target polynucleotide sequence wherein the complementary extender probe sequence contains at least one thiophosphate and does not terminate at the 3' terminus of the extender probe, and (b) treating the medium to cause hybridization of the extender probe to the single stranded target polynucleotide sequence, extension of the extender probe along the single stranded target polynucleotide sequence, and degradation of the 3' terminus of the extender probe.

In another embodiment the presence of a target polynucleotide sequence in a medium suspected of containing the target polynucleotide sequence is detected. The target polynucleotide sequence has two non-contiguous, non-hybridizable nucleotide sequences, S1 and S2, wherein S2 is 5' of S1 and at least 10 nucleotides long. The method comprises the steps of:
(a) providing in combination, either concomitantly or wholly or partially sequentially, (1) the medium, (2) an extender probe having two deoxynucleotide sequences wherein the sequence at the 3'-end of the extender probe (EP1) is hybridizable with S1 and the other of the deoxynucleotide sequences (EP2) is substantially identical to S2, (3) means for modifying the 3'-end of the extender probe not hybridized with the target polynucleotide sequence, (4) a polydeoxynucleotide primer capable of hybridizing with a nucleotide sequence complementary to S2 with the proviso that the primer may be generated in situ, (5) DNA polymerase and (6) deoxynucleoside triphosphates under conditions wherein (A) extender probe hybridizes with and is extended along (extended EP) the target polynucleotide sequence to form a duplex, (B) extender probe not hybridized to the target polynucleotide sequence is modified at its 3'-end, (C) the extended EP is dissociated from the duplex, (D) the primer hybridizes with and is extended along the extended EP to form a second duplex comprising extended primer, (E) the extended primer is dissociated from the duplex, and (F) the primer hybridizes with and is extended along the extended primer to form a duplex comprising extended primer and steps (E) and (F) are repeated, and
(b) examining for the presence of the extended primer.

Another embodiment of the invention involves a method for detecting the presence of a polynucleotide analyte in a sample suspected of containing the polynucleotide analyte. The method comprises the steps of:
(a) treating a medium containing the sample to form a single stranded target polynucleotide sequence from the polynucleotide analyte, if present, the target polynucleotide sequence having two non-contiguous, non-complementary nucleotide sequences, S1 and S2, wherein S2 is 5' of S1, and is at least ten nucleotides long,
(b) combining the medium with (1) an extender probe having two polydeoxynucleotide sequences wherein the sequence at the 3'-end of the extender probe (EP1) is hybridizable with S1 and the other of the deoxynucleotide sequence (EP2) is substantially identical to S2, (2) a nucleotide sequence (NS) having a portion capable of hybridizing with EP1 wherein NS may be a separate molecule or part of the extender probe, (3) a polydeoxynucleotide primer capable of hybridizing with a nucleotide sequence complementary to S2 when means for degrading said extender probe to form said polydeoxynucleotide primer is not present, (4) deoxynucleoside triphosphates, and (5) DNA template dependent polydeoxynucleotide polymerase under conditions wherein (A) extender probe is hybridized with and is extended along (extended extender probe) the target polynucleotide sequence to form a duplex, (B) extender probe not hybridized to the target polynucleotide sequence is extended along NS, (C) the extended extender probe is dissociated from the duplex, (D) the primer hybridizes with and is extended along the extended extender probe to form a duplex comprising extended primer, (E) the extended primer is dissociated from the duplex, and (F) the primer hybridizes with and is extended along the extended primer to form a duplex comprising extended primer, and steps (E) and (F) are repeated, wherein steps (a) and (b) are performed concomitantly or wholly or partially sequentially, and
(c) examining for the presence of the extended primer.

Another embodiment of the invention concerns a method for detecting the presence of a polynucleotide analyte in a sample suspected of containing the polynucleotide analyte. The method comprises the steps of:
(a) treating a medium containing the sample to form a single stranded target polynucleotide sequence from the polynucleotide analyte, if present, the target polynucleotide sequence having two non-contiguous, non-complementary nucleotide sequences S1 and S2 wherein S2 is 5' of S1, and is at least ten nucleotides long,
(b) combining the medium with (1) an extender probe having two deoxynucleotide sequences wherein the sequence at the 3'-end of the extender probe (EP1) is hybridizable with S1 and the other of the deoxynucleotide sequences (EP2) is substantially identical to S2 and not complementary to the target polynucleotide sequence, (2) an enzyme capable of degrading single stranded polynucleotides, (3) a polydeoxynucleotide primer capable of hybridizing with a nucleotide sequence complementary to S2 when means for degrading said extender probe to form said polynucleotide primer is not present, (4) deoxynucleoside triphosphates, and (5) DNA template dependent polydeoxynucleotide polymerase under conditions wherein (A) the extender probe is hybridized with and is extended along the target polynucleotide sequence to form a duplex, (B) extender probe not hybridized to the target polynucleotide sequence is degraded, (C) the extended extender probe is dissociated from the duplex, (D) the primer hybridizes with and is extended along the extended extender probe, (E) the extended primer is dissociated from the duplex, and (F) the primer hybridizes with and is extended along the extended primer to form a duplex comprising extended primer and steps (E) and (F) are repeated, wherein steps (a) and (b) are performed concomitantly or wholly or partially sequentially, and
(c) examining for the presence of the extended primer.

The invention further includes kits comprising in packaged combination (a) a polydeoxynucleotide extender probe having at its 3'-end a sequence (EP1) hybridizable with a first sequence in a target polynucleotide sequence and having a sequence (EP2) that is substantially identical to a second sequence of the target polynucleotide sequence, wherein in the target polynucleotide sequence the second sequence is 5' of, and non-contiguous with, the first sequence, (b) means for modifying the 3'-end of extender probe not hybridized with the target polynucleotide sequence, and (c) a polydeoxynucleotide primer capable of hybridizing with a sequence that is complementary with the second sequence.

### Brief Description of the Drawings

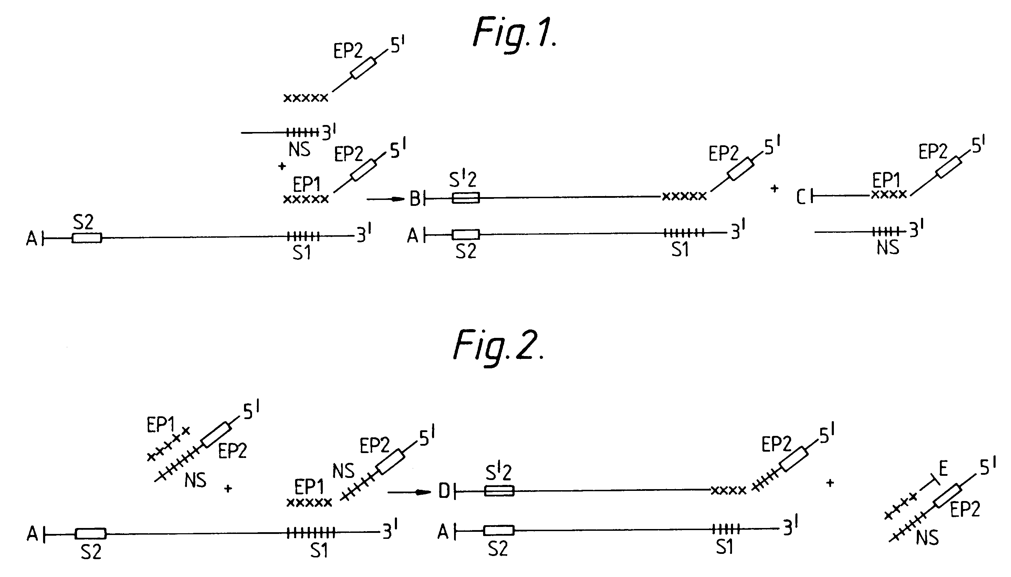

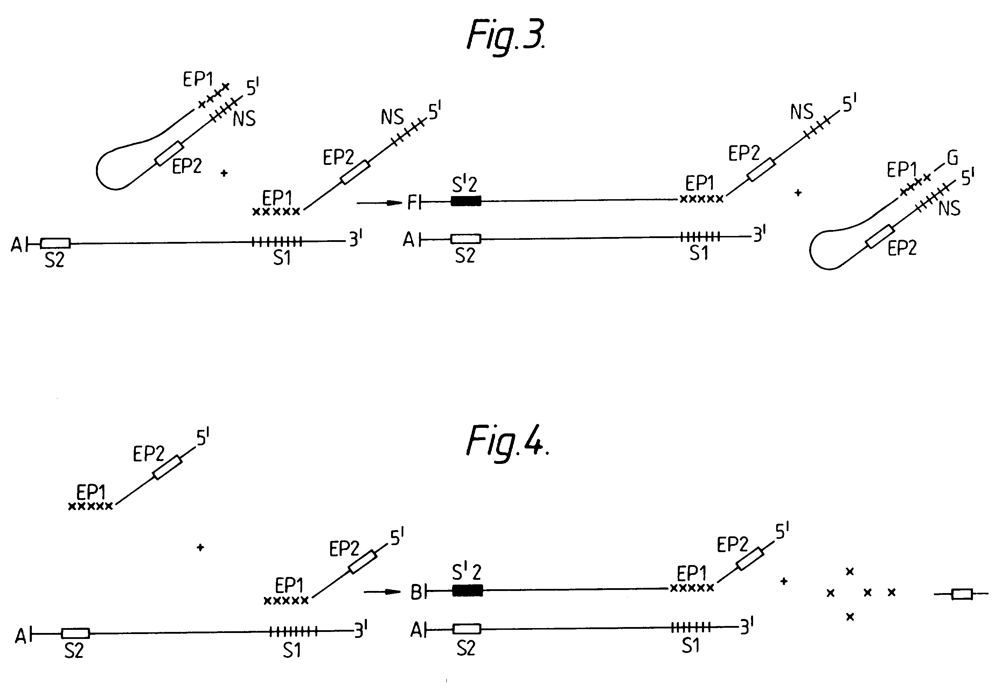

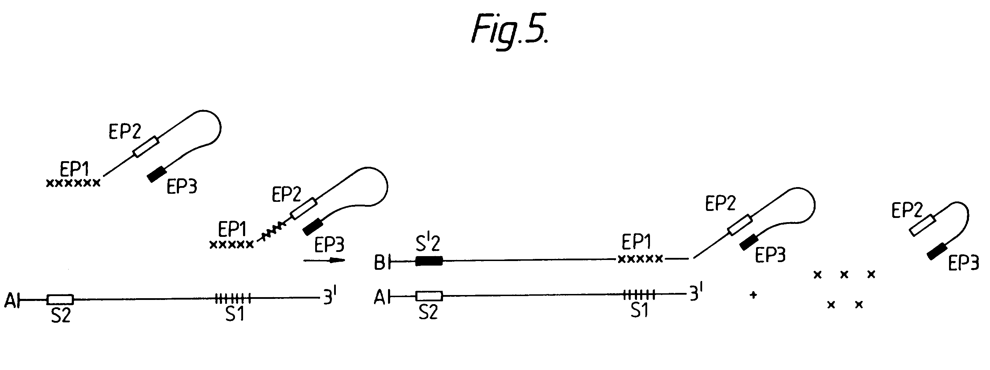

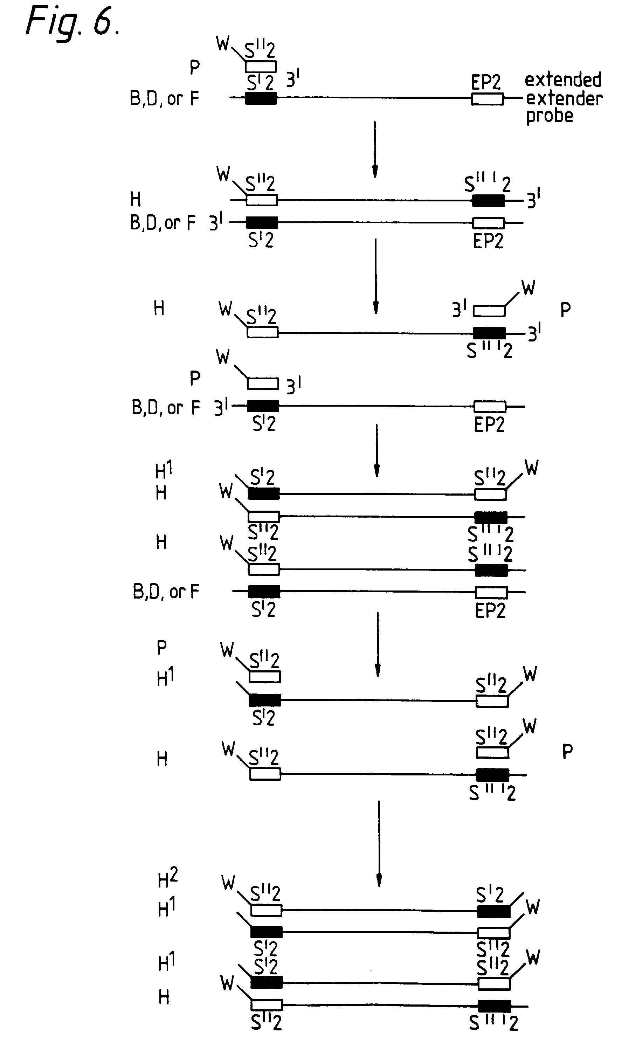

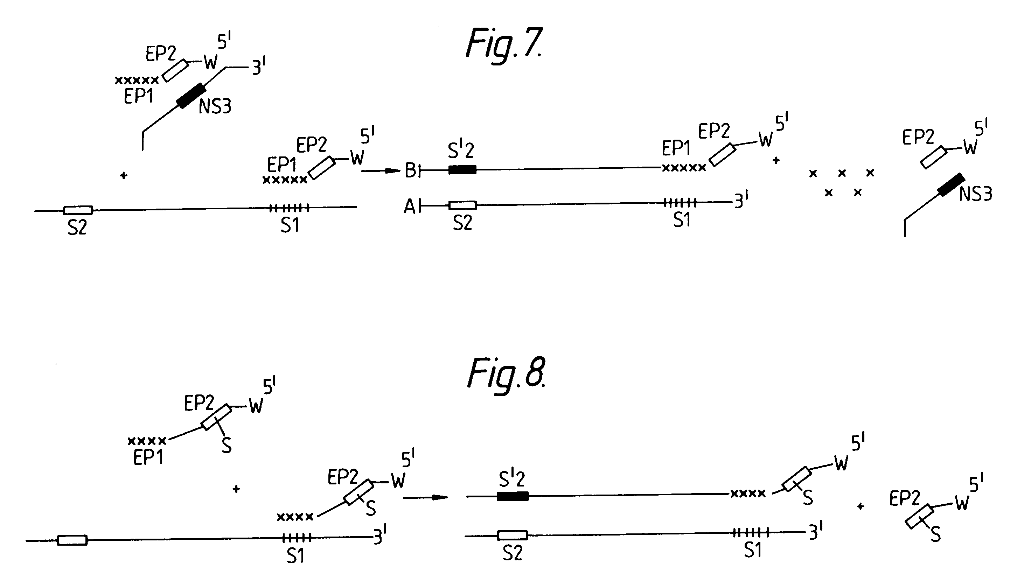

Figs. 1-8 are schematics of different embodiments in accordance with the present invention.

### Description of the Specific Embodiments

The present method allows extension of an extender probe along single stranded (ss) target polynucleotide sequence to produce a single stranded polynucleotide having the capability of forming an intramolecularly base-paired structure wherein the 3'-end of extender probe not involved in the production of the single stranded polynucleotide is modified. The single stranded polynucleotide produced in this manner can have an intramolecularly base-paired structure, i.e., two segments that are non-contiguous and complementary with each other, sometimes referred to as an inverted repeat. The method has particular application in the area of single primer amplification described above, in which a target polynucleotide sequence in a sample is amplified when such target polynucleotide sequence has an inverted repeat or can be converted to such a structure. The present method provides a highly convenient method for converting a polynucleotide sequence of interest to a target polynucleotide sequence having an intramolecularly base-paired structure while minimizing the number of reagents and steps required.

In its broadest aspect the present invention provides for production of a single stranded polynucleotide sequence having an inverted repeat that is formed from an extender probe, wherein all the extender probe not hybridized to a target polynucleotide sequence is modified at its 3'-end and is accordingly not present in unmodified form in the medium containing the newly formed single stranded polynucleotide. A target polynucleotide sequence is combined in a medium with an extender probe comprising (1) a sequence at the 3'-end of the extender probe that is complementary to a first sequence at the 3'-end of a target polynucleotide sequence within the target polynucleotide sequence and (2) a second sequence of the extender probe that is substantially identical to a second sequence of the target polynucleotide sequence, wherein each of said second sequences is 5' of each of said first sequences. The medium is treated to cause hybridization of the extender probe to the target polynucleotide sequence, along which the extender probe is extended, and to cause degradation of the 3'-end of the extender probe not hybridized to the target polynucleotide sequence.

Before proceeding further with a description of the specific embodiments of the present invention, a number of terms will be defined.

Polynucleotide analyte--a compound or composition to be measured that is a polymeric nucleotide, which in the intact natural state can have about 20 to 500,000 or more nucleotides and in an isolated state can have about 30 to 50,000 or more nucleotides, usually about 100 to 20,000 nucleotides, more frequently 500 to 10,000 nucleotides. It is thus obvious that isolation of the analyte from the natural state often results in fragmentation. The polynucleotide analytes include nucleic acids from any source in purified or unpurified form including DNA (dsDNA and ssDNA) and RNA, including t-RNA, m-RNA, r-RNA, mitochondrial DNA and RNA, chloroplast DNA and RNA, DNA-RNA hybrids, or mixtures thereof, genes, chromosomes, plasmids, the genomes of biological material such as microorganisms, e.g., bacteria, yeasts, viruses, viroids, molds, fungi, plants, animals, humans, and fragments thereof, and the like. The polynucleotide analyte can be only a minor fraction of a complex mixture such as a biological sample. The analyte can be obtained from various biological material by procedures well known in the art. Some examples of such biological material by way of illustration and not limitation are disclosed in Table I below.

**Table I**

| Microorganisms of interest include: | | |
|---|---|---|
| Corynebacteria | | |
| Corynebacterium diphtheria | | |
| Pneumococci | | |
| Diplococcus pneumoniae | | |
| Streptococci | | |
| Streptococcus pyrogenes | | |
| Streptococcus salivarus | | |
| Staphylococci | | |
| Staphylococcus aureus | | |
| Staphylococcus albus | | |
| Neisseria | | |
| Neisseria meningitidis | | |
| Neisseria gonorrhea | | |
| Enterobacteriaciae | | |
| Escherichia coli | | |
| Aerobacter aerogenes | | The colliform |
| Klebsiella pneumoniae | | bacteria |
| Salmonella typhosa | | |
| Salmonella choleraesuis | | The Salmonellae |
| Salmonella typhimurium | | |
| Shigella dysenteria | | |
| Shigella schmitzii | | |
| Shigella arabinotarda | | |
| | | The Shigellae |
| Shigella flexneri | | |
| Shigella boydii | | |
| Shigella sonnei | | |
| Other enteric bacilli | | |
| Proteus vulgaris | | |
| Proteus mirabilis | | Proteus species |
| Proteus morgani | | |
| Pseudomonas aeruginosa | | |
| Alcaligenes faecalis | | |
| Vibrio cholerae | | |
| Hemophilus-Bordetella group | | Rhizopus oryzae |
| Hemophilus influenza, H. ducryi | | Rhizopus arrhizua Phycomycetes |
| Hemophilus hemophilus | | Rhizopus nigricans |
| Hemophilus aegypticus | | Sporotrichum schenkii |
| Hemophilus parainfluenza | | Flonsecaea pedrosoi |
| Bordetella pertussis | | Fonsecacea compact |
| Pasteurellae | | Fonsecacea dermatidis |
| Pasteurella pestis | | Cladosporium carrionii |
| Pasteurella tulareusis | | Phialophora verrucosa |
| Brucellae | | Aspergillus nidulans |
| Brucella melitensis | | Madurella mycetomi |
| Brucella abortus | | Madurella grisea |
| Brucella suis | | Allescheria boydii |
| | | |
| Aerobic Spore-forming Bacilli | | Phialophora jeanselmei |
| Bacillus anthracis | | Microsporum gypseum |
| Bacillus subtilis | | Trichophyton mentagrophytes |
| Bacillus megaterium | | Keratinomyces ajelloi |
| Bacillus cereus | | Microsporum canis |
| Anaerobic Spore-forming Bacilli | | Trichophyton rubrum |
| Clostridium botulinum | | Microsporum adouini |
| Clostridium tetani | | Viruses |
| Clostridium perfringens | | Adenoviruses |
| Clostridium novyi | | Herpes Viruses |
| Clostridium septicum | | Herpes simplex |
| Clostridium histolyticum | | Varicella (Chicken pox) |
| Clostridium tertium | | Herpes Zoster (Shingles) |
| Clostridium bifermetans | | Virus B |
| Clostridium sporogenes | | Cytomegalovirus |
| Mycobacteria | | Pox Viruses |
| Mycobacterium tuberculosis | | Variola (smallpox) |
| hominis | | |
| Mycobacterium bovis | | Vaccinia |
| Mycobacterium avium | | Poxvirus bovis |
| Mycobacterium leprae | | Paravaccinia |
| Mycobacterium paratuberculosis | | Molluscum contagiosum |
| Actinomycetes (fungus-like bacteria) | | Picornaviruses |
| Actinomyces Isaeli | | Poliovirus |
| Actinomyces bovis | | Coxsackievirus |
| Actinomyces naeslundii | | Echoviruses |
| Nocardia asteroides | | Rhinoviruses |
| Nocardia brasiliensis | | Myxoviruses |
| The Spirochetes | | Influenza(A, B, and C) |
| Treponema pallidum | Spirillum minus | Parainfluenza (1-4) |
| Treponema pertenue | Streptobacillus | Mumps Virus |
| | monoiliformis | Newcastle Disease Virus |
| Treponema carateum | | Measles Virus |
| Borrelia recurrentis | | Rinderpest Virus |
| Leptospira icterohemorrhagiae | | Canine Distemper Virus |
| Leptospira canicola | | Respiratory Syncytial Virus |
| Trypanasomes | | Rubella Virus |
| Mycoplasmas | | Arboviruses |
| Mycoplasma pneumoniae | | |
| Other pathogens | | Eastern Equine Eucephalitis |
| Virus | | |
| Listeria monocytogenes | | Western Equine Eucephalitis |
| Virus | | |
| Erysipelothrix rhusiopathiae | | Sindbis Virus |
| Streptobacillus moniliformis | | Chikugunya Virus |
| Donvania granulomatis | | Semliki Forest Virus |
| Bartonella bacilliformis | | Mayora Virus |
| Rickettsiae (bacteria-like parasites) | | St. Louis Encephalitis Virus |
| Rickettsia prowazekii | | California Encephalitis Virus |
| Rickettsia mooseri | | Colorado Tick Fever Virus |
| Rickettsia rickettsii | | Yellow Fever Virus |
| Rickettsia conori | | Dengue Virus |
| Rickettsia australis | | Reoviruses |
| Rickettsia sibiricus | | Reovirus Types 1-3 |
| | | Retroviruses |
| Rickettsia akari (HIV) | | Human Immunodeficiency Viruses |
| Rickettsia tsutsugamushi | | Human T-cell Lymphotrophic Virus I & II (HTLV) |
| Rickettsia burnetti | | Hepatitis |
| Rickettsia quintana | | Hepatitis A Virus |
| Chlamydia (unclassifiable parasites bacterial/viral) | | Hepatitis B Virus |
| | | Hepatitis nonA-nonB Virus |
| Chlamydia agents (naming uncertain) | | Tumor Viruses |
| Fungi | | Rauscher Leukemia Virus |
| Cryptococcus neoformans | | Gross Virus |
| Blastomyces dermatidis | | Maloney Leukemia Virus |
| Hisoplasma capsulatum | | |
| Coccidioides immitis | | Human Papilloma Virus |
| Paracoccidioides brasiliensis | | |
| Candida albicans | | |
| Aspergillus fumigatus | | |
| Mucor corymbifer (Absidia corymbifera) | | |

The polynucleotide analyte, where appropriate, may be treated to cleave the analyte to obtain a fragment that contains a target polynucleotide sequence, for example, by shearing or by treatment with a restriction endonuclease or other site specific chemical cleavage method. However, it is an advantage of the present invention that the polynucleotide analyte can be used in its isolated state without further cleavage.

For purposes of this invention, the polynucleotide analyte, or a cleaved fragment obtained from the polynucleotide analyte, will usually be at least partially denatured or single stranded or treated to render it denatured or single stranded. Such treatments are well-known in the art and include, for instance, heat or alkali treatment. For example, double stranded DNA can be heated at 90-100° C. for a period of about 1 to 10 minutes to produce denatured material.

Target polynucleotide sequence -- a sequence of nucleotides to be identified, usually existing within a polynucleotide analyte, the identity of which is known to an extent sufficient to allow preparation of an extender probe polydeoxynucleotide that will hybridize with at least a portion of such target sequence, usually at least a ten nucleotide segment at the 3'-end thereof and preferably at least 15, frequently 20 to 50 nucleotide segment thereof and that comprises at least a ten nucleotide segment substantially identical to the 5'-end thereof. The target polynucleotide sequence has two non-contiguous, non-complementary nucleotide sequences, S1 and S2, one of which (S1) is the aforesaid portion capable of hybridizing to an extender probe polydeoxynucleotide wherein S2 is 5' of S1. The target polynucleotide sequence usually will contain from about 30 to 5,000 or more nucleotides, preferably 50 to 1,000 nucleotides. The two non-contiguous, non-complementary nucleotide sequences, S1 and S2, preferably contain from 10 to 100 nucleotides each and are separated by at least ten bases, preferably at least 100, usually 200 to 10,000. One target polynucleotide sequence is frequently a part of the polynucleotide analyte. The target polynucleotide sequence will generally be a fraction of a larger molecule or it may be substantially the entire molecule. The minimum number of nucleotides in the target polynucleotide sequence will be selected to assure that the presence of target polynucleotide sequence in a sample will be a specific indicator of the presence of polynucleotide analyte in a sample. Very roughly, the sequence length will usually be greater than about 1.6 log L nucleotides where L is the number of base pairs in the genome of the biologic source of the sample. The maximum number of nucleotides in the target sequence will normally be governed by the length of the polynucleotide analyte and its tendency to be broken by shearing, or other processes during isolation and any procedures required to prepare the sample for assay and the efficiency of detection and/or amplification of the sequence.

Single stranded polydeoxynucleotide sequence -- a sequence of deoxynucleotides that is formed as a result of the present invention. It will normally be comprised at least of two segments or flanking sequences that are non-contiguous and complementary with each other. It may also contain one or more sequences which, when bound to their complementary sequences, are specific binding sites for receptors such as repressors, restriction enzymes, and the like. The first and second segments or flanking sequences are at the 3'-end and 5'-end, respectively, of the single stranded polynucleotide sequence and each comprises at least ten, preferably at least 15, deoxynucleotides, and/or derivatives thereof.

The single stranded polydeoxynucleotide sequence will usually contain from 30 to 10,000 deoxynucleotides, preferably 100 to 2,000 deoxynucleotides, more preferably 500 to 5,000 deoxynucleotides. When the single stranded polydeoxynucleotide sequence is hybridized with a complementary strand, each end will have a member of a pair of inverted repeats.

Polydeoxynucleotide primer -- a polydeoxynucleotide, usually a synthetic deoxynucleotide that is single stranded, containing a sequence at its 3'-end that is identical with the sequence S2 or hybridizable with a nucleotide sequence complementary with the sequence S2 of the target polynucleotide sequence. Normally the polydeoxynucleotide primer will have at least 90%, preferably 100%, of the same basic sequence as the second nucleotide sequence EP2 of the extender probe. The number of deoxynucleotides in the hybridizable sequence of polydeoxynucleotide primer should be such that stringency conditions used to hybridize the polydeoxynucleotide primer will prevent excessive random non-specific hybridization. Usually, the number of deoxynucleotides in the polydeoxynucleotide primer will be at least as great as in the S2 sequence of the target polynucleotide sequence, namely, at least ten deoxynucleotides, preferably at least 15 deoxynucleotides and generally from about 10 to 200, preferably 20 to 50, deoxynucleotides.

Deoxynucleoside triphosphates -- a deoxynucleoside having a 5'-triphosphate substituent. The deoxynucleosides are pentose sugar derivatives of nitrogenous bases of either purine or pyrimidine derivation, covalently bonded to the 1'-carbon of the pentose sugar. The purine bases include adenine(A), guanine(G), inosine, and derivatives and analogs thereof. The pyrimidine bases include cytosine (C), thymine (T), uracil (U), and derivatives and analogs thereof.

The derivatives and analogs are exemplified by those that are recognized and polymerized in a similar manner to the underivitized nucleoside triphosphates. Examples of such derivatives or analogs by way of illustration and not limitation are those which are modified with a reporter group, biotinylated, amine modified, radiolabeled, alkylated, and the like and also include phosphorothioate, phosphite, ring atom modified derivatives, and the like. The reporter group can be a fluorescent group such as fluoroscein, a chemiluminescent group such as luminol, a terbium chelator such as N-(hydroxyethyl) ethylenediaminetriacetic acid that is capable of detection by delayed fluorescence, and the like.

Polydeoxynucleotide polymerase -- a catalyst, usually an enzyme, for forming an extension of the polydeoxynucleotide primer along a DNA template including the single stranded polydeoxynucleotide where the extension is complementary thereto. The polydeoxynucleotide polymerase is a template dependent polydeoxynucleotide polymerase and utilizes the deoxynucleoside triphosphates as building blocks for extending the 3'-end of the polydeoxynucleotide primer to provide a sequence complementary with the single stranded polydeoxynucleotide sequence. Usually, the catalysts are enzymes, such DNA polymerases such as, for example, prokaryotic DNA polymerase (I, II, or III), T4 DNA polymerase, T7 DNA polymerase, Klenow fragment, reverse transcriptase, and the like, derived from any source such as cells, bacteria, such as E. coli, plants, animals, virus, thermophilic bacteria, and so forth. Where the polynucleotide or target polynucleotide sequence is RNA, reverse transcriptase would be included to facilitate extension of the extender probe along the polynucleotide or target polynucleotide sequence.

Wholly or partially sequentially -- when the sample and various agents utilized in the present invention are combined other than concomitantly (simultaneously), one or more may be combined with one or more of the remaining agents to form a subcombination. Each subcombination can then be subjected to one or more steps of the present method. Thus, each of the subcombinations can be incubated under conditions to achieve one or more of the desired results.

Hybridization (hybridizing) and binding--in the context of nucleotide sequences these terms are used interchangeably herein. The ability of two nucleotide sequences to hybridize with each other is based on the degree of complementarity of the two nucleotide sequences, which in turn is based on the fraction of matched complementary nucleotide pairs. The more nucleotides in a given sequence that are complementary to another sequence, the more stringent the conditions can be for hybridization and the more specific will be the binding of the two sequences. Increased stringency is achieved by elevating the temperature, increasing the ratio of cosolvents, lowering the salt concentration, and the like.

Homologous or substantially identical--In general, two polynucleotide sequences that are identical or can each hybridize to the same polynucleotide sequence are homologous. The two sequences are homologous or substantially identical where the sequences each have at least 90%, preferably 100%, of the same or analogous base sequence where thymine (T) and uracil (U) are considered the same. Thus, the ribonucleotides A, U, C and G are taken as analogous to the deoxynucleotides dA, dT, dC, and dG, respectively. Homologous sequences can both be DNA or one can be DNA and the other RNA.

Complementary--Two sequences are complementary when the sequence of one can bind to the sequence of the other in an anti-parallel sense wherein the 3'-end of each sequence binds to the 5'-end of the other sequence and each A, T(U), G, and C of one sequence is then aligned with a T(U), A, C, and G, respectively, of the other sequence.

Extender probe--is a single polynucleotide strand, usually a synthetic oligonucleotide, comprised of two sequences of nucleotides, one of such sequences (EP1) located at the 3'-end of the strand, being a deoxynucleotide sequence having preferably at least ten consecutive deoxynucleotides and capable of hybridizing with a first polynucleotide sequence (S1) of the target polynucleotide sequence.

The major criteria for choosing EP1 are: (1) The sequence should be reliable, that is, it should be closely or exactly complementary with S1 and should be of sufficient length to provide stable and specific binding. (2) The 3'-end must have, or be capable of forming, a free 3'-hydroxyl group. The minimum length of EP1 will usually be at least 10, normally at least 15, preferably 20-50, deoxynucleotides. In general, EP1 will be about 20 to 100 deoxynucleotides. The combined length of the first and second polynucleotide sequences of the extender probe is at least about 20 nucleotides, preferably about 40 to 200 nucleotides, in length.

The second polynucleotide sequence of the extender probe (EP2) is a sequence of nucleotides substantially identical or homologous to the second polynucleotide sequence (S2) of a target polynucleotide sequence. EP2 is at least 10 nucleotides, usually at least 15, preferably 20-50 deoxynucleotides, in length. In general EP2 will be about 20 to 100 deoxynucleotides.

The extender probe may contain additional receptor binding or spacer sequences or other sequences located between EP1 and EP2 or at the end of EP2.

Non-contiguous--sequences are non-contiguous, there being at least one usually at least 10 nucleotides present in the target polynucleotide sequence between the two segments or between two sequences, S1 and S2, of a polynucleotide.

Contiguous--sequences are considered to be contiguous when there are no nucleotides between two segments or between two sequences of a polynucleotide.

Copy -- means a sequence that is a direct identical or homologous copy of a single stranded polynucleotide sequence as differentiated from a sequence that is complementary to the sequence of such single stranded polynucleotide. In single primer amplification conducted in conjunction with the present invention, a complementary sequence of a single stranded polydeoxynucleotide sequence is produced initially as the result of the extension of the polydeoxynucleotide primer, and a sequence that is a direct copy of the single stranded polydeoxynucleotide sequence is subsequently obtained from the aforementioned complementary sequence.

Means for extending an extender probe--an extender probe having an extendable 3'-terminus can be extended by combining the extender probe hybridized to a polynucleotide, such as a target polynucleotide sequence, with a polydeoxynucleotide polymerase and deoxynucleoside triphosphates under conditions for extending the extender probe. In this way the extender probe is extended along the polynucleotide to form a duplex. When extension occurs along the target polynucleotide sequence, the duplex is comprised of the extended extender probe. Extension in this fashion provides the requisite fidelity between the two strands so that subsequent amplification of the extended extender probe provides accurate detection of the target of interest.

Means for extending a primer--a polydeoxynucleotide primer having an extendable 3'-terminus can be extended by combining the primer hybridized to extended extender probe or extended primer with a polydeoxynucleotide polymerase and deoxynucleoside triphosphates under conditions for extending the primer. In this way the primer is extended along the extended extender probe or extended primer to form a duplex comprising the extended primer. Extension in this fashion provides the requisite fidelity between the extended primer and the polynucleotide so that accurate detection of target analytes can be achieved.

Means for modifying the 3'-end of the extender probe--for single primer amplification as described above complementary base sequences in a single polynucleotide strand capable of forming a stem loop structure or inverted repeat are utilized. Such polynucleotide is either present in a sample or is created in response to the presence of a polynucleotide analyte. An extender probe is utilized to create such a polynucleotide by virtue of binding to a target polynucleotide, along which the extender probe is extended. Since the concentration of polynucleotide analyte is generally low and unknown, there are molecules of extender probe that do not hybridize with the target polynucleotide sequence. These molecules of extender probe are undesirable because they might result in competing processes, which reduce the efficiency of single primer amplification. By employing appropriate means the 3'-end of extender probe not bound to a target polynucleotide sequence can be modified such that it can no longer be extended along the target polynucleotide sequence in the presence of deoxynucleoside triphosphates and DNA polymerase.

One way in which the 3'-end of the extender probe can be modified is by degradation. For example, an enzyme such as an 3'-exonuclease can be added to the reaction medium. Under certain conditions such an enzyme degrades the 3'-end of single stranded polynucleotides. Examples of such exonuclease enzymes, by way of illustration and not limitation, are Klenow fragment, T4 polymerase, and T7 polymerase. In one approach, the polydeoxynucleotide polymerase, such as DNA polymerase, utilized for the extension of the extender probe has exonuclease activity. Exemplary of such DNA polymerases are Klenow, T4 and T7 DNA polymerases.

In another embodiment the 3'-end of the extender probe is extended along a scavenger polynucleotide that has a sequence NS at other than its 5'-end, said sequence being hybridizable with the 3'-end of the extender probe. When the 3'-terminus of the extender probe and the scavenger polynucleotide sequence are hybridized, the 3'-end of the extender probe can be extended along the scavenger polynucleotide sequence in the presence of polydeoxynucleotide polymerase and deoxynucleoside triphosphates. This process results in modification of the 3'-end of the extender probe, thereby rendering the extender probe incapable of extension along the target polynucleotide sequence or its complement during single primer amplification. The scavenger polynucleotide sequence is, generally, 8 to 1,000 or more nucleotides, preferably 10 to 50 nucleotides, in length and may be part of the extender probe or a molecule separate from the extender probe. When the scavenger polynucleotide sequence is part of the extender probe, it may be 3' or 5' of the sequence EP2 of the extender probe. Enzymes that can be utilized in this chain extension are commercially available thermophilic nucleotide polymerases such as, by way of example and not limitation, Taq, Vent, Hot Tub and the like.

Member of a specific binding pair ("sbp member")--one of two different molecules, having an area on the surface or in a cavity which specifically binds to and is thereby defined as complementary with a particular spatial and polar organization of the other molecule. The members of the specific binding pair are referred to as ligand and receptor (antiligand). These may be members of an immunological pair such as antigen-antibody, or may be operator-repressor, nuclease-nucleotide, biotin-avidin, hormones-hormone receptors, nucleic acid duplexes, IgG-protein A, DNA-DNA, DNA-RNA, and the like.

Ligand--any compound for which a receptor naturally exists or can be prepared.

Receptor ("antiligand")--any compound or composition capable of recognizing a particular spatial and polar organization of a molecule, e.g., epitopic or determinant site. Illustrative receptors include naturally occurring receptors, e.g., thyroxine binding globulin, antibodies, enzymes, Fab fragments, lectins, nucleic acids, repressors, protection enzymes, protein A, complement component C1q, DNA binding proteins or ligands and the like.

Small organic molecule--a compound of molecular weight less than 1500, preferably 100 to 1000, more preferably 300 to 600 such as biotin, fluorescein, rhodamine and other dyes, tetracycline and other protein binding molecules, and haptens, etc. The small organic molecule can provide a means for attachment of a nucleotide sequence to a label or to a support.

Support or surface--a porous or non-porous water insoluble material. The support can be hydrophilic or capable of being rendered hydrophilic and includes inorganic powders such as silica, magnesium sulfate, and alumina; natural polymeric materials, particularly cellulosic materials and materials derived from cellulose, such as fiber containing papers, e.g., filter paper, chromatographic paper, etc.; synthetic or modified naturally occurring polymers, such as nitrocellulose, cellulose acetate, poly (vinyl chloride), polyacrylamide, cross linked dextran, agarose, polyacrylate, polyethylene, polypropylene, poly(4-methylbutene), polystyrene, polymethacrylate, poly(ethylene terephthalate), nylon, poly(vinyl butyrate), etc.; either used by themselves or in conjunction with other materials; glass available as Bioglass, ceramics, metals, and the like. Natural or synthetic assemblies such as liposomes, phospholipid vesicles, and cells can also be employed.

Binding of sbp members to the support or surface may be accomplished by well-known techniques, commonly available in the literature. See, for example, "Immobilized Enzymes," Ichiro Chibata, Halsted Press, New York (1978) and Cuatrecasas, J. Biol. Chem., 245:3059 (1970). The surface can have any one of a number of shapes, such as strip, rod, particle, including bead, and the like.

Label or reporter group or reporter molecule--a member of the signal producing system. Usually the label or reporter group or molecule is conjugated to or becomes bound to a polynucleotide probe or a polydeoxynucleotide primer and is capable of being detected directly or, through a specific binding reaction, and can produce a detectible signal. Labels include a polynucleotide primer or specific polynucleotide sequence that can provide a template for amplification or ligation or act as a ligand such as for a repressor protein. Preferably, the polydeoxynucleotide primer will have, or be capable of having, a label. In general, any label that is detectable can be used. The label can be isotopic or nonisotopic, usually non-isotopic, and can be a catalyst, such as an enzyme, a polynucleotide coding for a catalyst, promoter, dye, fluorescent molecule, chemiluminescer, coenzyme, enzyme substrate, radioactive group, a small organic molecule, amplifiable polynucleotide sequence, a particle such as latex or carbon particle, metal sol, crystallite, liposome, cell, etc., which may or may not be further labeled with a dye, catalyst or other detectible group, and the like. The label is a member of a signal producing system and can generate a detectable signal either alone or together with other members of the signal producing system. The label can be bound directly to a nucleotide sequence or can become bound thereto by being bound to an sbp member complementary to an sbp member that is bound to a nucleotide sequence.

Signal Producing System--The signal producing system may have one or more components, at least one component being the label or reporter group. The signal producing system generates a signal that relates to the presence or amount of target polynucleotide sequence or a polynucleotide analyte in a sample. The signal producing system includes all of the reagents required to produce a measurable signal. When the label is not conjugated to a nucleotide sequence, the label is normally bound to an sbp member complementary to an sbp member that is bound to or part of a nucleotide sequence. Other components of the signal producing system may be included in a developer solution and can include substrates, enhancers, activators, chemiluminescent compounds, cofactors, inhibitors, scavengers, metal ions, specific binding substances required for binding of signal generating substances, and the like. Other components of the signal producing system may be coenzymes, substances that react with enzymic products, other enzymes and catalysts, and the like. The signal producing system provides a signal detectable by external means, by use of electromagnetic radiation, desirably by visual examination. The signal-producing system is described more fully in EP469755 (U.S. Patent Application Serial No. 07/555,323, filed July 19, 1990).

Ancillary Materials--Various ancillary materials will frequently be employed in the assay in accordance with the present invention. For example, buffers will normally be present in the assay medium, as well as stabilizers for the assay medium and the assay components. Frequently, in addition to these additives, proteins may be included, such as albumins, organic solvents such as formamide, quaternary ammonium salts, polycations such as dextran sulfate, surfactants, particularly non-ionic surfactants, binding enhancers, e.g., polyalkylene glycols, or the like.

In one aspect of the invention a method is provided for forming a single stranded polynucleotide sequence complementary to a single stranded target polynucleotide sequence wherein an extender probe is extended along the target and extender probe not hybridized to the target polynucleotide sequence is modified at its 3'-end.

One embodiment of the method is depicted schematically in Fig. 1. EP1, located at the 3'-end of the extender probe, hybridizes with S1 of the target polynucleotide sequence and with a portion of a scavenger polynucleotide containing sequence NS. EP2 is homologous with S2. The extender probe is extended along A to produce an extended extender probe B containing sequence S'2, which is complementary to S2. B now contains EP2 and S'2, which are hybridizable with each other. Extender probe not hybridized with the target polynucleotide sequence hybridizes with NS and is extended along the scavenger polynucleotide to produce a modified extender probe C, wherein EP1 is no longer located at a 3'-end. Preferably, NS is about 8 to 100, more preferably 8 to 30, nucleotides in length.

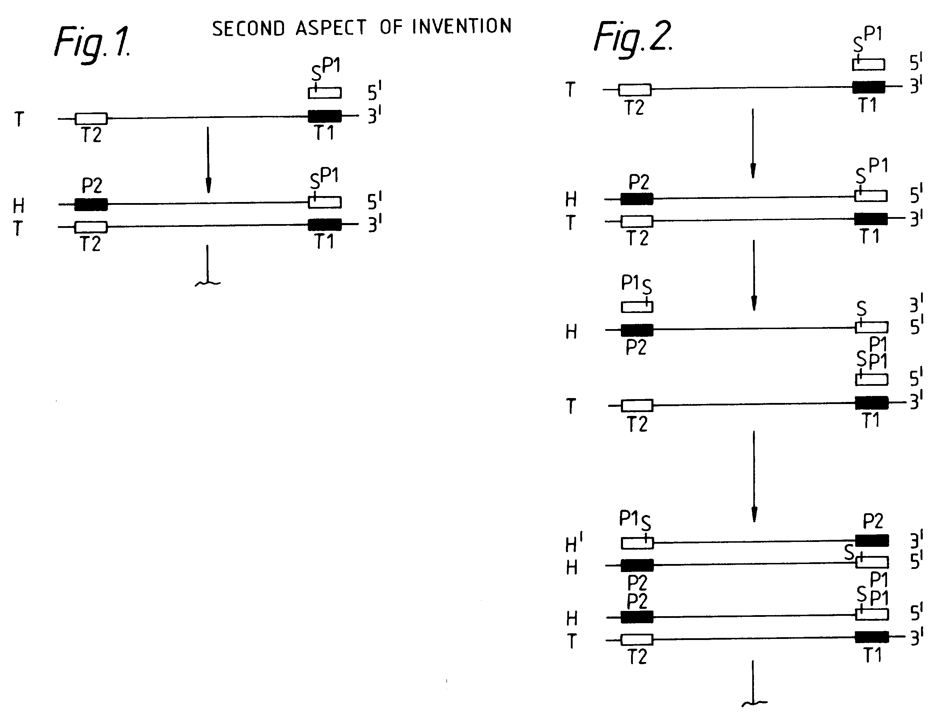

Another embodiment of the invention is depicted in Fig. 2. In this embodiment the extender probe contains not only EP1 and EP2, but also contains the sequence NS, which is 3' of sequence EP2. NS is homologous to S1. EP1, located at the 3'-end of the extender probe, hybridizes with S1 of the target polynucleotide sequence and with NS of the extender probe. EP2 is homologous with S2. The extender probe is extended along A to produce an extended extender probe D containing a sequence S'2, which is complementary to S2. D now contains EP2 and S'2, which are hybridizable with each other. Extender probe not hybridized with the target polynucleotide sequence loops back on and hybridizes with itself, EP1 hybridizing with NS. The 3'-end of this extender probe is extended along itself to produce a modified extender probe E, wherein EP1 is no longer located at a 3'-end.

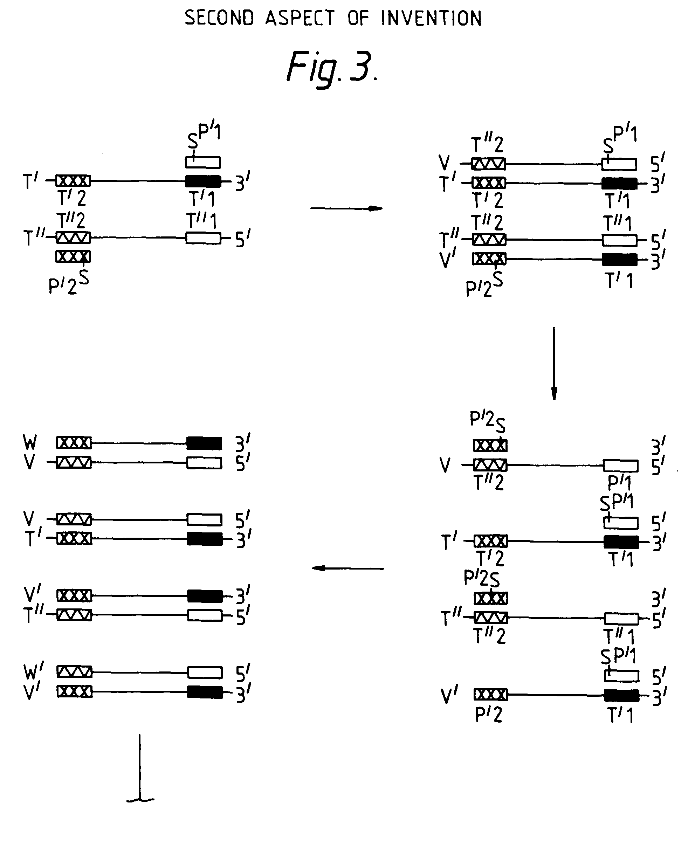

Another alternate embodiment is shown in Fig. 3, the sequence NS is contained in the extender probe 5' of EP2. Hybridization and extension of the extender probe with the target polynucleotide sequence and with itself take place as described above for the embodiment of Fig. 2.

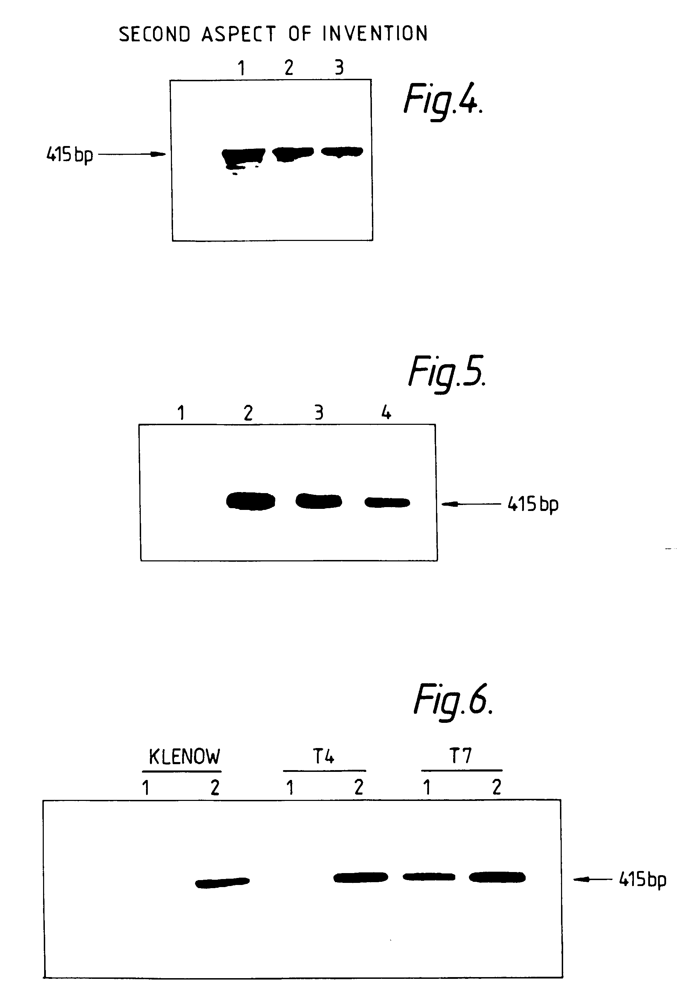

In another alternative embodiment of the present invention as depicted in Fig. 4, EP1 of the extender probe hybridizes with S1 of the target polynucleotide sequence. The extender probe is extended along A to produce extended extender probe B as described above for the embodiment of Fig. 1. Present in the reaction mixture is an exonuclease having 3' activity, which degrades extender probe not hybridized with A to an extent sufficient to destroy its ability to hybridize at its 3'-end with S1.

A variation of the embodiment of Fig. 4 is shown in Fig. 5. The extender probe contains not only EP1 and EP2 but also contains EP3, which is a sequence capable of hybridizing with EP2 and is preferably complementary with at least the 3'-end of EP2. EP1 of the extender probe hybridizes with S1 of the target polynucleotide sequence. Extended extender probe B is formed as described above for the embodiment of Fig. 4. The exonuclease degrades extender probe not bound to the target polynucleotide sequence back to its double strand formed by the hybridization of EP3 and EP2. The extender probe, in this embodiment, is designed such that its degradation removes at least EP1. Preferably, EP2 is about 5 to 50, more preferably, 8 to 30, nucleotides in length. The variant provides the option to use the degraded extender probe as a primer in a subsequent step in which EP2 of the degraded extender probe binds to S2 of the extended extender probe and extends along the extended extender probe.

The methods find use in single primer amplification wherein one or more copies of a target polynucleotide sequence, i.e., sequences identical to the target polynucleotide sequence, are formed free of any extender probe. Extender probe is hybridized to a target polynucleotide sequence and is extended as described above. Extender probe not bound to the target is modified at its 3'-end in any of the embodiments mentioned above. A polydeoxynucleotide primer is then hybridized at least at its 3'-end with a nucleotide sequence complementary to S2 under conditions where (1) the extended extender probe is rendered single stranded, (2) the polydeoxynucleotide primer hybridizes with and is extended along the extended extender probe to form a duplex comprising extended primer, which contains a sequence identical to the target polynucleotide sequence. Preferably, the concentration of the extender probe is substantially lower than that of the polydeoxynucleotide primer. By "substantially lower" is meant that the concentration of extender probe relative to primer is at least 1 to 10, usually 1 to 100 or more. Preferably, the concentration of the extender probe is less than one percent that of the polydeoxynucleotide primer.

The use of the present method in single primer amplification is depicted in Fig. 6.

Polydeoxynucleotide primer P has a sequence at its 3'-end (S"2) that hybridizes with S'2, wherein S'2 is complementary to S2 of the target polynucleotide sequence. Preferably, S''2 is a sequence identical to S2. P can also comprise a label W. P is hybridized with and extended along extended extender probe B (Fig. 1), D (Fig. 2) or F (Fig. 3), (which has been dissociated from its duplex) to form extended primer H comprising sequences S''2 and S"'2, S"'2 is complementary to EP2 and preferably identical to S'2. B, D or F and H are dissociated and P hybridizes with S"'2 of H and S'2 of B, D or F and P is extended along B, D or F and H to yield H and H¹, respectively. H¹ has complementary sequences S'2 and S''2. The duplexes are dissociated and P is hybridized with and extended along H¹ and H to yield H¹ and H². Further repetition results in multiple copies of H¹ and H², which can be detected because of the presence of label W.

In one embodiment of the invention the present method can be utilized to modify the 3'-end of the extender probe and form the polydeoxynucleotide primer in situ. This embodiment is depicted in Fig. 7. The extender probe contains EP1 and EP2, wherein EP2 is equivalent to primer sequence S''2 and optionally may contain a label W. EP1 of the extender probe hybridizes with S1 of the target polynucleotide sequence and with a sequence within a scavenger polynucleotide, NS3, which is complementary with at least a portion of EP2. S2 is homologous with EP2. The extender probe is extended along A to produce an extended extender probe B containing sequence S'2, which is complementary to S2. B now contains EP2 and S'2, which are hybridizable with each other. Extender probe hybridized to NS3 is degraded by an exonuclease having 3' activity, which is added to the reaction medium. The extender probe is constructed such that its degradation produces polydeoxynucleotide primer P, which is utilized in single primer amplification. Accordingly, NS3 hybridizes with EP2, at least at its 3'-end, so that EP1 is degraded by the exonuclease leaving EP2 at the 3'-end of the remaining polynucleotide. Reaction conditions are chosen such that further degradation is impeded by the presence of a double strand formed by NS3 hybridized to EP2.

Another convenient approach to control degradation of the extender probe so as to produce the polydeoxynucleotide primer in situ is depicted in Fig. 8. It involves the use of a 3'-exonuclease and one or more phosphorothioate diesters (indicated in Fig. 8 by - S) in place of phosphate diesters between the ultimate and penultimate nucleosides at the 3'-end of EP2. Degradation of extender probe that is not bound to the target polynucleotide sequence will stop at the phosphorothioate diester or one or more nucleotides 3' of said phosphorothioate. The degraded extender probe has the sequence EP2 at its 3'-end with a phosphorothioate near the 3'-end and functions as a primer P to chain extend in accordance with single primer amplification. In this embodiment EP2 and S''2 are identical.

When the present method is applied to replicating a target polynucleotide sequence, one of the above described embodiments is followed and the following steps are repeated at least once: (a) the polydeoxynucleotide primer is caused to hybridize with and extend along the extended extender probe to form a second duplex comprising extended primer and (b) the extended primer is dissociated from the second duplex. Normally this process will be repeated at least three times whereupon the primer also is hybridized with and is extended along the extended primer to form a duplex comprising the extended primer which is thereupon dissociated. Preferably, at least a fifteen nucleotide sequence EP1 of the extender probe hybridizes with S1. Preferably, also, the polydeoxynucleotide primer contains at least a fifteen deoxynucleotide sequence S''2 capable of hybridizing with a sequence complementary to S2.

Preferably, S1 and S2 each respectively contain from 10 to 100 nucleotides. The method has application where the target polynucleotide sequence is DNA or RNA. In one aspect the polydeoxynucleotide primer is labeled with a reporter molecule. The reporter molecule can be, for example, a detectable group or a binder such as biotin or a nucleotide sequence other than the sequence that hybridizes with the sequence complementary to S2. The extended primer can be detected by means of a reporter molecule covalently bonded to a probe. The probe will usually have a nucleotide sequence that is homologous or complementary to a portion of the target nucleotide sequence other than S1 or S2.

Another embodiment of the invention concerns a method for detecting the presence of a polynucleotide analyte in a sample suspected of containing the polynucleotide analyte. A medium containing the sample is treated as described above to form a single stranded target polynucleotide sequence from the polynucleotide analyte, if present. The target polynucleotide sequence has two non-contiguous, non-complementary nucleotide sequences S1 and S2 wherein S2 is 5' of S1, and is at least ten nucleotides long. The medium is combined with an extender probe having two deoxynucleotide sequences. The sequence at the 3'-end of the extender probe (EP1) is hybridizable with S1. The other of the deoxynucleotide sequences (EP2) is homologous to S2. Means for modifying the 3'-end of extender probe not hybridized with the target nucleotide sequence is included. A polydeoxynucleotide primer capable of hybridizing with a nucleotide sequence complementary to S2 is included when modification of the extender probe does not provide a primer. Deoxynucleoside triphosphates and one or more polydeoxynucleotide polymerases are also combined. Conditions are chosen such that (1) the extender probe is hybridized with and is extended along the target polynucleotide sequence to form a duplex, (2) the extender probe not hybridized with the target polynucleotide sequence is modified, (3) the extended extender probe is dissociated from the duplex, (4) the primer hybridizes with and is extended along the extended sequence to form a second duplex comprising extended primer, (5) the extended primer is dissociated from the duplex, and (6) the primer hybridizes with and is extended along said extended primer to form a duplex comprising extended primer. Steps (5) and (6) are repeated and steps (a) and (b) are performed concomitantly or wholly or partially sequentially. Then, an examination is conducted for the presence of the extended primer, the presence thereof indicating the presence of the polynucleotide analyte. Steps (5) and (6) are repeated a least three times, preferably, at least 10 times; usually it is preferable that the number of repetitions be less than 30. Generally, steps (5) and (6) are repeated a number of times sufficient to provide an accurate detection of the polynucleotide analyte. Where the polynucleotide analyte is RNA, the polydeoxynucleotide polymerase comprises a reverse transcriptase.

In carrying out the method of forming the single stranded polydeoxynucleotide using an extender probe, modifying the extender probe not hybridized to a target polynucleotide sequence and the amplification, an aqueous medium will be employed. Other polar cosolvents may also be employed, usually oxygenated organic solvents of from 1-6, more usually from 1-4, carbon atoms, including alcohols, ethers and the like. Usually these cosolvents will be present in less than about 70 weight percent, more usually in less than about 30 weight percent.

The pH for the medium will usually be in the range of about 4.5 to 9.5, more usually in the range of about 5.5 - 8.5, and preferably in the range of about 6 - 8. The pH and temperature are chosen and varied, as the case may be, so as to cause, either simultaneously or sequentially, dissociation of any internally hybridized sequences, hybridization of the extender probe with the target polynucleotide sequence and any other sequence that forms part of the means for modifying the 3'-end of the extender probe, hybridization of the polydeoxynucleotide primer with extended extender probe and extended primer, extension of the extender probe and primer, degradation of the 3'-end of the extender probe by an exonuclease, dissociation of the extended extender probe and extended primer. In some instances, a compromise will be made in optimizing the speed, efficiency, and specificity of these steps depending on whether it is desired to perform the above steps performed sequentially or simultaneously. Various buffers may be used to achieve the desired pH and maintain the pH during the determination. Illustrative buffers include borate, phosphate, carbonate, Tris, barbital and the like. The particular buffer employed is not critical to this invention but in individual methods one buffer may be preferred over another.

Moderate temperatures are normally employed for carrying out the method. Normally, in conducting the method the medium will be cycled between two or three temperatures. The temperatures for the method will generally range from about 10 to 105°C, more usually from about 40 to 99°C, preferably 50 to 98°C. The exact temperatures can be varied depending on the salt concentration, pH, solvents used, length of the target and S1 and S2 sequences and composition of the target polynucleotide sequence and the primer. Relatively low temperatures of from about 30 to 65°C can be employed for the extension steps, while denaturation and hybridization can be carried out at a temperature of from about 50 to 105°C. Degradation of the 3'-end of the extender probe by an exonuclease is usually conducted at a temperature of about 15 to 100°C, preferably 20 to 50°C.

The time period for carrying out the modification of the 3'-end of the extender probe not hybridized to a target polynucleotide sequence will generally be about 0.5 to 30 minutes, preferably 1 to 20 minutes. Where the present method is utilized in single primer amplification, the method is conducted for a time sufficient to achieve a desired number of copies of the extended primer or a sequence complementary thereto. This, in turn, depends on the purpose for which the amplification is conducted, such as, for example, an assay for a polynucleotide analyte. Generally, the time period for conducting the method will be from about 1 to 10 minutes per cycle and any number of cycles can be used from 1 to as high as 200 or more, usually 5 to 80, frequently 10-60. As a matter of convenience it will usually be desirable to minimize the time period and the number of cycles. In general, the time period for a given degree of amplification can be shortened, for example, by selecting concentrations of nucleoside triphosphates sufficient to saturate the polynucleotide polymerase and by increasing the concentrations of polynucleotide polymerase and polynucleotide primer. Generally, the time period for conducting the method will be from about 5 to 200 minutes. As a matter of convenience, it will usually be desirable to minimize the time period.

The above conditions may also be chosen for forming a target polynucleotide sequence from a polynucleotide analyte.

The amount of reagents for modifying the 3'-end of the extender probe varies depending on the particular means for achieving the modification. In the situation wherein modification involves extension of the 3'-end of the extender probe, the concentration of the template dependent polynucleotide polymerase and the deoxynucleotide triphosphates will generally be equal or more than that described below for the amplification and may require a different enzyme. The concentration of reagents utilized for the extension of the extender probe along the target polynucleotide sequence and amplification will be sufficient to extend the extender probe not bound to the target polynucleotide sequence. The concentration of any scavenger polynucleotide sequence will generally be at least as great as the concentration of extender probe and usually at least 10-fold higher.

Where modification of the extender probe is accomplished by means of a 3' exonuclease, the concentration of the exonuclease is selected to degrade the extender probe to the desired extent in a practical time period such as 0.5-20 minutes. Preferably, the template dependent polynucleotide polymerase will also have 3' exonuclease activity, and, thus, the concentration of this polymerase will be chosen to be sufficient to accomplish chain extension and degradation. Usually, when the 3'-end of the extender probe is to be completely degraded, the magnesium ion concentration in the initial enzyme reaction is kept low (less than 4mM, for example) and the pH remains high (greater than 8.0, for example).

The concentration of the extender probe, as mentioned above, can be substantially less than that of the primer. Preferably, the extender probe concentration is less than one percent of that of the primer, more preferably less than 0.1% that of the primer usually the extender probe concentration will be less than 1 nmolar, frequently less than 0.1 nmolar (nM) whereas the primer concentration will usually be greater than 10 nmolar, usually at least 100 nmolar. Preferably, the concentration of primer is greater than 100 nM while that of the extender probe is less than 1 nM.

The amount of the target polynucleotide sequence which is to be copied can be as low as one or two molecules in a sample but will generally vary from about 10² to 10¹⁰, more usually from about 10³ to 10⁸ molecules in a sample preferably at least 10⁻²¹M in the sample and may be 10⁻¹⁰ to 10⁻¹⁹M, more usually 10⁻¹⁴ to 10⁻¹⁹M. The amount of the polydeoxynucleotide primer will be at least as great as the number of copies desired and will usually be 10⁻¹³ to 10⁻⁸ moles per sample, where the sample is 1-1,000 µL. Usually, the primer will be present in at least 10⁻⁹ M, preferably 10⁻⁷ M, and more preferably at least about 10⁻⁶ M. Preferably, the concentration of the polynucleotide primer is substantially in excess over, preferably at least 100 times greater than, the concentration of the single stranded polynucleotide.

The concentration of the deoxynucleoside triphosphates in the medium can vary widely; preferably, these reagents are present in an excess amount. The deoxynucleoside triphosphates will usually be present in 10⁻⁶ to 10⁻²M, preferably 10⁻⁵ to 10⁻³M.

The concentration of the template-dependent polynucleotide polymerase will usually be determined empirically. Preferably, a concentration will be used that is sufficient such that further increase in the concentration will not decrease the time for the amplification by over 5-fold, preferably 2-fold. The primary limiting factor generally is the cost of the reagent.

The order of combining of the various reagents to form the combination may vary. Generally, the target polynucleotide sequence is obtained from a sample containing such sequence or a polynucleotide analyte that has been treated to obtain such sequence. Generally, the target polynucleotide sequence and the extender probe are combined with a pre-prepared combination of any polynucleotide sequence needed for modification of the 3'-end of the extender probe not bound to the target polynucleotide sequence, deoxynucleoside triphosphates, template-dependent polydeoxynucleotide polymerase and a 3' exonuclease where appropriate. Where needed, a polynucleotide primer may be included in the prepared combination or may be added subsequently. However, simultaneous addition of all of the above, as well as other step-wise or sequential orders of addition, may be employed.

The concentration and order of addition of reagents and conditions for the method are governed generally by the desire to maximize the number of copies of the extended primer and the rate at which such copies are formed and the fidelity of replication. Generally, it is desirable to increase the number of copies of the extended primer by at least a factor of 10², preferably a factor of 10⁴, more preferably 10⁶ or more.

In carrying out the method of the invention as applied to the detection of a polynucleotide analyte, the considerations as to media, pH, temperature, and times can be as described above.

While the concentrations of the various reagents will generally be determined by the concentration range of interest of the polynucleotide analyte, the final concentration of each of the reagents will normally be determined empirically to optimize the sensitivity of the assay over the range of interest. The concentration of the other reagents in an assay generally will be determined following the same principles as set forth above for the amplification method. The primary consideration is that a sufficient number of copies of extended primer be produced, free of any extender probe, in relation to the polynucleotide analyte sequence so that such copies can be readily detected and provide an accurate determination of the polynucleotide analyte.

The copies of extended primer can be detected in numerous ways. For example, in the present method, molecules of the polydeoxynucleotide primer can be labeled with a reporter molecule such as a ligand, a small organic molecule, a polynucleotide sequence, a protein, support, a member of an operator-repressor pair, intercalation dye and the like.

Examples of particular labels or reporter molecules and their detection can be found in U.S. Patent Application Serial No. 07/555,323 filed July 19, 1990, see also EP469755.

Other assay formats and detection formats are disclosed in U.S. Patent Applications Serial Nos. 07/299,282 and 07/399,795 filed January 19, 1989, and August 29, 1989, respectively, see also EP379369.

Any standard method for specifically detecting nucleic acid sequences can be used.

One method for detecting nucleic acids is to employ nucleic acid probes.

One method utilizing probes is described in U.S. Patent Application Serial No. 773,386, filed September 6, 1985, US4868104.

Detection of the signal will depend upon the nature of the signal producing system utilized. If the label or reporter group is an enzyme, additional members of the signal producing system would include enzyme substrates and so forth. The product of the enzyme reaction is preferably a luminescent product, or a fluorescent or non-fluorescent dye, any of which can be detected spectrophotometrically, or a product that can be detected by other spectrometric or electrometric means. If the label is a fluorescent molecule the medium can be irradiated and the fluorescence determined. Where the label is a radioactive group, the medium can be counted to determine the radioactive count.

Various techniques can be employed for preparing an extender probe, polydeoxynucleotide primer, or other polynucleotide sequences utilized in the present invention. They can be obtained by biological synthesis or by chemical synthesis. For short sequences (up to about 100 nucleotides) chemical synthesis will frequently be more economical as compared to the biological synthesis. In addition to economy, chemical synthesis provides a convenient way of incorporating low molecular weight compounds and/or modified bases during the synthesis step. Furthermore, chemical synthesis is very flexible in the choice of length and region of the target polynucleotide binding sequence. The extender probe, polydeoxynucleotide primer and other polynucleotides can be synthesized by standard methods such as those used in commercial automated nucleic acid synthesizers. Chemical synthesis of DNA on a suitably modified glass or resin can result in DNA covalently attached to the surface. This may offer advantages in washing and sample handling. For longer sequences standard replication methods employed in molecular biology can be used such as the use of M13 for single stranded DNA as described by J. Messing (1983) Methods Enzymol, 101, 20-78.

Other methods of oligonucleotide synthesis include phosphotriester and phosphodiester methods (Narang et al. (1979) Meth. Enzymol 68: 90) and synthesis on a support (Beaucage, et al. (1981) Tetrahedron Letters 22: 1859-1862) as well as phosphoramidate technique, Caruthers, M. H., et al., "Methods in Enzymology," Vol. 154, pp. 287-314 (1988), and others described in "Synthesis and Applications of DNA and RNA," S.A. Narang, editor, Academic Press, New York, 1987, and the references contained therein.

Extender probes containing at least one phosphorothioate diester can be prepared according to known techniques. Oligonucleotide synthesis can be carried out as described above up to the point where introduction of the phosphorothioate diester is desired. The phosphorothioate diester can be introduced in a number of ways such as, for example, oxidations utilizing a thiolating reagent such as a diacyldisulfide or tetraethyl thiuram disulfide, which are commercially available. The remaining nucleotides are then introduced. Other methods of preparing phosphorothioate containing polynucleotides are described in WO9008838, WO8911486, U.S. Patent No. 4,910,300, EP318245, the relevant disclosures of which are incorporated herein by reference. Other methods of preparing a phosphorothioate containing polynucleotide are described by (a) Yau, et al., Tetrahedron Lett. (1990)31(14): 1953-1956; (b) Brill, et al., ibid. (1989) 30(48):6621-6624; (c) Caruthers, et al., Nucleic Acids Symp. Ser. (1989)21: 119-120; (d) Caruthers, et al., Nucleosides Nucleotides (1988)8(5-6): 1011-1014; (e) Brill, et al., J. Am. Chem. Soc. (1989)111(6): 2321-2322.

In some instances, the 3'-end of a polynucleotide will be modified to prevent reaction with template dependent DNA polymerase or to append a binding sequence. The 3'-end can, for example, be modified by ligation of a dideoxynucleotide or a ribonucleotide followed by oxidation of the ribose with periodate followed by reductive amination of the resulting dialdehyde with borohydride and a bulky amine such as aminodextran.

As a matter of convenience, predetermined amounts of reagents employed in the present invention can be provided in a kit in packaged combination. In assaying for a polynucleotide analyte in a sample, a kit useful in the present method can comprise, in packaged combination with other reagents, reagents for forming a target polynucleotide sequence from a polynucleotide analyte, an extender probe having at its 3'-end a sequence hybridizable with a first sequence in a target polynucleotide sequence and having a sequence that is homologous to a second sequence of the target polynucleotide sequence, wherein the second sequence is 5' and non-contiguous with the first sequence, and a polydeoxynucleotide primer, the latter of which can be labeled or can be provided with groups to render the sequence labeled or bound to a support. The kit can further include a labeled polynucleotide probe capable of binding to the target polynucleotide sequence, any polynucleotide sequences necessary for modifying the 3'-end of extender probe not hybridized to the target polynucleotide sequence and also, where appropriate, a 3' exonuclease. The kits above can further include in the packaged combination deoxynucleoside triphosphates such as deoxynucleoside triphosphates, e.g., deoxyadenosine triphosphate (dATP), deoxyguanosine triphosphate (dGTP), deoxycytidine triphosphate (dCTP) and deoxythymidine triphosphate (dTTP). For use in a method of producing multiple copies, the kit can contain a polydeoxynucleotide primer if the primer is not produced by degradation of the extender probe. The kit can further include a polydeoxynucleotide polymerase and members of a signal producing system and also various buffered media, some of which may contain one or more of the above reagents.

The relative amounts of the various reagents in the kits can be varied widely to provide for concentrations of the reagents which substantially optimize the reactions that need to occur during the present method and to further substantially optimize the sensitivity of the assay. Under appropriate circumstances one or more of the reagents in the kit can be provided as a dry powder, usually lyophilized, including excipients, which on dissolution will provide for a reagent solution having the appropriate concentrations for performing a method or assay in accordance with the present invention. Each reagent can be packaged in separate containers or some reagents can be combined in one container where cross-reactivity and shelf life will permit.

### EXAMPLES

The invention is demonstrated further by the following illustrative examples. Temperatures are in degrees centigrade (°C) and parts and percentages are by weight, unless otherwise indicated.

### EXAMPLE 1

### Modification of Extender Probe by Exonuclease Degradation

Oligodeoxyribonucleotide sequences 1 and 2:
Polynucleotide extender probe;
Oligomer 1 
5' TGT TGT TCC GTT AGT TCG TTT TAT TTG TCG AAA TCC GCG
ACC TGC TCC ATG TTA CT3' (SEQ. No. 1), and
 Polydeoxynucleotide primer for amplification;
Oligomer 2 5' TGT TGT TCC GTT AGT TCG TTT TAT T 3' (SEQ. No. 2) were synthesized by the phosphoramidite method (Atkinson, T. and Smith, M. in Oliaonucleotide Synthesis: A Practical Approach, Gait, M.J. (ed.), IRL Press, Oxford, England (1984)) and purified on denaturing polyacrylamide gels according to standard procedures. The 25-5' terminal bases of extender probe oligomer 1 were identical to polydeoxynucleotide primer oligomer 2 and were used to generate an amplifiable polynucleotide sequence having an intramolecular base pair structure.

A protocol for DNA amplication of target polynucleotide bacteriophage M13mp19 (double-stranded replicative form, 7250 base pairs from Bethesda Research Laboratories) using oligomer 1 to form the initial amplifiable structure and subsequently oligomer 2 to drive the amplification was utilized. Ten picomoles (pmol) of oligomer 1 and 600 molecules of M13mp19 were combined in a buffer of 10 mM KCl, 10 mM (NH₄)₂SO₄, 20 mM Tris-CH1 (pH 8.8 @ 25°C), 2 mM MgSO₄, 0.1% Triton X-100, and 20 nanomoles (nmoles) of each dNTP. After the reaction mixture was denatured at 95°C for 5 minutes and cooled to room temperature to allow annealing of the extender probe oligomer 1 to the template, 5 units of T7 DNA polymerase (New England Biolabs), 5 units of T4 DNA polymerase (Bethesda Research Laboratories) or 4 units of Klenow fragment (U.S. Biochemical) was added to the reaction and incubated at 37°C for 5-10 minutes. During this incubation, any extender probe oligomer 1, which was not annealed to the target polynucleotide was degraded by the 3' to 5' exonuclease activity of the above enzymes. Any extender probe which annealed to the target polynucleotide was extended by the polymerase activity of the enzymes to form the amplifiable polynucleotide having an intramolecular base paired structure. The T7, T4, or Klenow polymerase was then heat inactivated by incubation at 95°C for 2 minutes and the mixture was again cooled to room temperature. 100 pmoles of oligomer 2 and 1 to 2 units of Vent DNA polymerase (New England Biolabs) were then added for a final volume of 100 microliters (µl). Temperature cycling of 90°C (30 seconds), 55°C (1 minute), and 72°C (5 minutes for the first 10 cycles and 1.5 minutes thereafter) was performed using a programmable thermal cycler (Ericomp, Inc.) for a number of cycles through the above three temperatures. Aliquots from these reactions were withdrawn at the conclusion of temperature cycling and were analyzed by electrophoresis through 1.2% agarose (Seakem GTG, FMC BioProducts) gels in 1X TAE buffer [40 mM Tris-Acetate (pH 10.3 @ 23°C), 10 mM EDTA] and the DNA products were visualized by staining the gel with ethidium bromide.

In order to confirm that the treatment with the 3' to 5' exonuclease completely removed any extender probe oligomer 1 not annealed to the target polynucleotide from the reaction, a trace amount of extender oligomer 1 labeled at the 5'-end with 32P using T4 polynucleotide kinase (USB) was included in the reaction. An aliquot was removed after the exonuclease incubation and analyzed by denaturing polyacrylamide gel electrophoresis followed by autoradiography. Results obtained from this experiment are summarized in Table 1.

**TABLE 1**

| 3' to 5' exonuclease treatment | Target DNA | Extender probe present after cycle 1 | Amplification after 60 cycles |
|---|---|---|---|
| - | none | + | - |
| + | none | - | - |
| - | 600 molecules | + | + |
| + | 600 molecules | - | + |

The results in Table 1 demonstrate that treatment of the reaction mixture with a DNA polymerase which possesses a 3' to 5' exonuclease activity, after annealing of the extender probe 1 oligomer to the target polynucleotide but before the addition of the amplification polydeoxynucleotide primer and the thermostable DNA polymerase, completely removed all of the extender probe oligomer 1 from the reaction and permitted the formation of enough polynucleotide having an intramolecular base paired structure to allow amplification from 600 double-stranded DNA targets.

### EXAMPLE 2

### Modification of Extender Probe by Chain Extension

Oligodeoxyribonucleotide sequences 1 and 2:
Polynucleotide extender probe;
Oligomer 1 
5' TAG CTA GCA GTA ACA TGG AGC AGT GTT GTT CCG TTA GTT
CGT TTT ATT TGT CGA AAT CCG CGA CCT GCT CCA TGT TAC T 3'
(SEQ. No. 3), and
 Polydeoxynucleotide primer;
Oligomer 2 5' TGT TGT TCC GTT AGT TCG TTT TAT T 3' (SEQ. No. 4) were synthesizeed by the phosphoramidite method and purified on denaturing polyacrylamide gels. Contained within oligomer 1 is the entire sequence of oligomer 2 (bases 24-48), which were used to generate an amplifiable polynucleotide having an intramolecular base paired structure. Bases 9-23 and 65-79 of oligomer 1 comprise an inverted repeat, which is capable of forming an intramolecular base paired structure consisting of a 15 basepair stem and a 41 base loop. The eight 5' terminal bases of oligomer 1 are not complementary to the target polynucleotide, i.e., bacteriophage M13mp19.

A protocol for DNA amplification of bacteriophage M13mp19 (double-stranded replicative form, 7250 base pairs) using extender probe oligomer 1 to form the initial polynucleotide having an intramolecular base paired structure and subsequently oligomer 2 to drive the amplification was utilized. Ten picomoles (pmol) of oligomer 1, 200 pmoles of oligomer 2, and 600 molecules of M13mp19 were combined in a buffer of 10mM KCl, 10 mM (NH₄)₂SO₄, 20 mM Tris-HCl (pH 8.8 @ 25°C), 2 mM MgSO₄, 0.1% Triton® X-100, and 20 nanomoles (nmoles) of each dNTP. The reaction mixture was denatured at 95°C for 5 minutes and cooled to room temperature to allow annealing of extender probe oligomer 1 to the target DNA. One to two units of Vent DNA polymerase (New England Biolabs) was then added for a final volume of 100 microliters (µl). The reactions were then incubated at 72°C for 10 minutes. During this step any extender probe oligomer 1 that annealed to the target polynucleotide was extended by Vent polymerase to form an intramolecular base paired (bp) structure, which was amplified by oligmer 2. Any extender probe oligomer 1 not annealed to the target polynucleotide formed an intramolecular stem-loop containing a 15 bp stem and an 8 base 5' single-stranded overhang. This 5' overhang was filled in by Vent polymerase with the complementary nucleotides. Since the 8 bases filled in by Vent polymerase were not complementary to the target polynucleotide, the modified extender probe oligomer 1 was rendered inactive as a primer. Any of this extender probe that may still anneal to the target has an 8 base mismatch at the 3'-end and thus can not be extended by Vent polymerase.

Temperature cycling of 90°C (30 seconds), 55°C (1 minute), and 72°C (5 minutes for the first 10 cycles and 1.5 minutes thereafter) was performed using a programmable thermal cycler (Ericomp, Inc.) for a number of cycles through the above three temperatures. Aliquots from these reactions were withdrawn at the conclusion of temperature cycling and were analyzed by electrophoresis through 1.2% agarose (Seakem GTG, FMC BioProducts) gels in 1XTAE buffer [40 mM Tris-Acetate (pH 10.3 @ 23°C), 10 mM EDTA] and the DNA products were visualized by staining the gel with ethidium bromide.

In order to confirm that all unannealed extender oligomer probe 1 was filled in by the action of Vent polymerase using deoxynucleotide triphosphates during the initial incubation at 72°C, a trace amount of extender probe oligomer 1 that had been labeled at the 5'-end with ³²P using T4 polynucleotide kinase (USB) was included in the reaction. An aliquot was removed after the initial incubation at 72°C and analyzed by denaturing polyacrylamide gel electrophoresis followed by autoradiography. Results obtained from this experiment are summarized in Table 2:

**TABLE 2**

| Target DNA | Fill-in of extender probe after cycle 1 | Amplification after 60 cycles |
|---|---|---|
| None | + | - |
| 600 molecules | + | + |

The results in Table 2 demonstrate that, in the presence of 600 double-stranded DNA targets, the extender probe oligomer formed an internal base paired structure or stem loop with a 5' single stranded overhang, which was effectively filled-in by Vent polymerase by the end of the first cycle of amplification, thereby preventing it from serving as a primer in subsequent rounds of amplification. The amplification primer then efficiently drove the amplification of the amplifiable intramolecular base paired structures that were formed in the initial cycle.

### EXAMPLE 3

### Modification of Extender Probe Utilizing a Phrosphorothioate- containing Oligonucleotide

The detection of approximately 600 double-stranded target molecules using single primer amplification was demonstrated repeatedly using a degradable, phosphorothioate-containing oligonucleotide. The oligonucleotide (56 bases) acts as the extender probe in creating an amplifiable stem-loop and, following nuclease treatment, serves as a primer to drive amplification.

The synthesis of the extender probe oligonucleotide was carried out in an automated (4-column Biosearch 8750 DNA synthesizer) manner until positioning of the thio-modified linkage(s). Manual oxidations were then performed with 0.1M tetraethyl thiuram disulfide (TETD) (Applied Biosystems, Inc., Foster City, CA) in acetonitrile. The remaining bases were added under normal coupling conditions following the protocol in Applied Biosystems, Inc., User Bulletin, Number 58, February 1991. In present example, two different extender probes, identical in sequence, differing only in the number and position of the phosphorothioate internucleotidic linkage(s) as seen below, were employed, one each in separate experiments.

The formation and amplification of a stem-loop molecule was carried out in 100 microliter reactions containing an appropriate buffer (20 mM Tris-HCl, pH 8.8, 10 mM KC1, 10 mM (NH₄)₂SO₄, 2 mM MgSO₄, and 0.1% Triton® X-100), dNTPs (200 to 300 micromolar), double stranded target polynucleotide molecule (600 M13mp19 molecules,), and the extender probe (0.5 to 4 micromolar initial concentration). Reactants were heat-denatured for 5 minutes at 95°C and annealed at 25°C (room temperature) for 15 to 20 minutes. A DNA polymerase with a strong 3' exonuclease activity was added (10 units per 100 µl of T7 DNA polymerase, New England Biolabs (NEB), Beverly Mass.). Reactions were incubated at 37°C such that any extender probe annealed to target was chain extended, whereas all non-annealed extender probe was degraded up to the position(s) of the thio linkage(s). The extender probe was radiolabelled at the 5'-end to monitor degradation. Complete degradation of the non-annealed extender probe up to the thio linkage(s) was obtained in as little as 1 minute, while the remaining sequence was resistant to further degradation for up to 15 minutes. After the elongation/degradation was complete, reactants were again heated at 95°C for 1 to 2 minutes, thereby inactivating the T7 polymerase and denaturing the newly formed stem-loop molecule from the original target molecule. A heat-stable polymerase was added (Pfu from Stratagene San Diego, CA, 5 units per 100 µl) and the reactions are cycled in a format as described in the previous examples. Aliquots from these reactions are analyzed by electrophoresis through 1.2% agarose (Seakem, FMC Bio Products) gels in 1 x TBE buffers [89 mM Tris-borate, 89 mM boric acid, 0.2 mM EDTA] and the amplified product was visualized by ethidium bromide staining.

Extender Probes containing thio linkage(s):
1. One phosphorothioate linkage (arrow) between A₃₄ and T₃₅:
   a) 23 base primer remained after degradation (underlined) 
3' TC ATT GTA CCT CGT CCA GCG CCT AAA GCT G T T T A↓T T
T T G C T T G A T TGCCTTGTTGT 5' (SEQ. No. 5)
2. Three phosphorothioate linkages (arrows) between T₃₃ and T₃₆:
   a) mixture of 3 primers (24, 23, 22 bases) remained after degradation (underlined)

3' TC ATT GTA CCT CGT CCA GCG CCT AAA GCT G T T T↓A↓T↓T
T T G C T T G A T TGCCTTGTTGT 5' (SEQ. NO. 6)

This invention also relates in a second aspect to the general use of the phosphorothioate-containing oligonucleotides as primers in nucleic acid amplification.

### SUMMARY OF THIS ASPECT OF THE INVENTION

In one embodiment of the present invention a method is described for forming a polynucleotide sequence complementary to a single stranded target polynucleotide sequence ("target sequence"). The method comprises the steps of (a) hybridizing to the 3'-end of the target sequence a polynucleotide primer having a 3'-terminus comprised of a nucleotide monophosphate in which at least one phosphate oxygen is replaced by sulfur, (b) extending the polynucleotide primer along the target sequence and (c) dissociating the extended polynucleotide primer from the target sequence.

Another embodiment of the present invention is a method for forming a polynucleotide sequence having a sequence identical, except for the presence of a sulfur bond to phosphorus in place of an oxygen, to a single stranded target polynucleotide sequence ("target sequence"). The method comprises the steps of (a) hybridizing to the 3'-end of the target sequence a first polynucleotide primer ("first primer") containing at least one phosphorous bond, (b) extending the first primer along the target sequence, (c) dissociating the extended first primer from the target sequence, (d) hybridizing to the 3'-end of the extended first primer a second polynucleotide primer ("second primer") wherein the second primer can be the same as or different from the first primer, and (e) extending the second primer along the extended first primer to form extended second primer wherein, when the first and second primers are the same, the extended first primer is a template for the first primer and, when the first and second primers are different, the extended first primer is a template for the second primer and the extended second primer is a template for the first primer.

The methods of the present invention have application to a methods for forming multiple copies of a target polynucleotide sequence. Generally, these methods comprise the step of forming extension products of a polynucleotide primer along the target sequence or along an extended polynucleotide primer where the extension products are copies of the target sequence. The improvement provided by the present invention comprises the primer having a 3'-terminus comprised of a nucleotide monophosphate containing at least one phosphorous-sulfur bond.

Accordingly, one embodiment of this aspect of the present invention is a method of producing multiple copies of a polynucleotide sequence. The method comprises (a) providing in combination (1) a single stranded polynucleotide having such polynucleotide sequence and being flanked at each end by at least partially complementary first and second flanking sequences, (2) a polynucleotide primer at least a 10 base portion of which at its 3'-end is hybridizable to that member of the first and second flanking sequences that is at the 3'-end of the single stranded polynucleotide, the polynucleotide primer containing at least one phosphorothioate linkage, (3) nucleoside triphosphates, (4) template dependent polynucleotide polymerase and (b) incubating the combination under conditions for either wholly or partially sequentially or concomitantly (1) dissociating the single stranded polynucleotide from any complementary sequences, (2) hybridizing the polynucleotide primer with the flanking sequence at the 3'-end of the single stranded polynucleotide, (3) extending the polynucleotide primer along the single stranded polynucleotide to provide a first extended polynucleotide primer, (4) dissociating the first extended primer and the single stranded polynucleotide, (5) hybridizing the first extended polynucleotide primer with the polynucleotide primer, (6) extending the polynucleotide primer along the first extended polynucleotide primer to provide a second extended polynucleotide primer, (7) dissociating the second extended polynucleotide primer from the first extended polynucleotide primer, and (8) repeating steps (5)- (7) above.

In accordance with this aspect of the present invention a method is available for forming multiple copies of a single stranded target polynucleotide sequence ("target sequence") containing at least one phosphorous-sulfur bond. The method comprises the steps of (a) hybridizing to the 3'-end of the target sequence a first polynucleotide primer ("first primer") containing at least one phosphorous-sulfur bond, (b) extending the first primer along the target sequence, the first primer being capable of hybridizing to, and being extended along, (1) extended first primer or (2) an extended second polynucleotide primer ("second primer") wherein the extended second primer results from the extension of a second primer capable of hybridizing to and extending along a sequence that is complementary (complementary sequence) to the target sequence, (c) dissociating extended first primer from the target sequence, (d) hybridizing to the 3'-end of the extended first primer the first or the second primer, (e) extending the first or the second primer along the extended first primer, (f) dissociating the extended first primer or the extended second primer from the extended first primer, (g) hybridizing to the 3'-end of the extended first or second primer the first primer, and (h) repeating steps (e)-(g).

Another embodiment of this aspect of the present invention is a method for forming multiple copies of at least one double stranded polynucleotide sequence ("polynucleotide sequence"). The sequence comprises a single stranded target polynucleotide sequence ("target sequence") and its complementary sequence. The method comprises the steps of (a) treating a sample suspected of containing one or more of the double stranded polynucleotide sequences with polynucleotide primers capable of hybridizing to each target and each complementary sequence suspected of being present in the sample under conditions for hybridizing the primers to, extending the primers along the target and the complementary sequences, wherein the primers are selected such that the extension product formed from one primer, when it is dissociated from its complement, can serve as a template for the formation of the extension product of another primer, wherein at least one of the primers for each of the double stranded polynucleotide sequences contains at least one phosphorous-sulfur bond, (b) dissociating the primer extension products from their templates if the sequence or sequences are present to produce single stranded molecules, and (c) treating the single stranded molecules produced in step (b) with the primers of step (a) under conditions such that a primer extension product is formed using the single strands produced in step (b) as templates, resulting in amplification of the target sequences and complementary sequences if present.

Another aspect of the present invention is a method for detecting the presence or absence of at least one specific polynucleotide sequence ("polynucleotide sequence") in a sample suspected of containing the polynucleotide sequence. The method comprises the steps of (a) treating a sample suspected of containing one or more of the polynucleotide sequences with one polynucleotide primer ("primer") for each polynucleotide sequence suspected of being present in the sample under hybridizing conditions such that for each different polynucleotide strand to which a primer is hybridized an extension product of each primer is formed which is complementary to each strand, wherein the primer or primers are selected so as to be sufficiently complementary to each strand to hybridize therewith such that the extension product formed from one primer, when it is dissociated from its complement, can serve as a template for the formation of the extension product of the other primer, wherein at least one of the primers contains at least one phosphorothioate linkage, (b) dissociating the primer extension products from their templates, if the sequence or sequences are present, to produce single stranded molecules, (c) treating the single stranded molecules produced in step (b) with the primers of step (a) under conditions such that a primer extension product is formed using each of the single strands produced in step (b) as a template, resulting in amplification of the target sequences and complementary sequences if present, (d) adding to the product of step (c) a labeled oligonucleotide probe for each sequence being detected capable of hybridizing to the sequence or a mutation thereof, and (e) determining whether such hybridization has occurred.

Another aspect of the present invention is a method for determining the presence of a polynucleotide analyte in a sample suspected of containing said analyte. The method comprises the steps of (a) forming as a result of the presence of the analyte a single stranded polynucleotide flanked by at least 80% complementary first and second flanking sequences, each comprised of at least 15 nucleotides, (b) forming in the presence of nucleoside triphosphates and template-dependent polynucleotide polymerase an extension of a polynucleotide primer at least the 3'-end of which can hybridize with the flanking sequence at the 3'-end of the single stranded polynucleotide sequence, wherein the polynucleotide primer contains at least one phosphorothioate linkage and wherein steps (a) and (b) can be performed wholly or partially sequentially or concominantly, and (c) detecting extended polynucleotide primer containing a sequence identical to and/or complementary with the polynucleotide sequence. The above method can involve a variation wherein in step (a) an extender probe is combined with the analyte, having two non-contiguous, non-complementary nucleotide sequences S1 and S2, wherein S2 is 5' of S1 and is at least 10 nucleotides long. The extender probe has a sequence S3 capable of hybridizing to S1 and having a sequence S4 that is 5' of S3 and homologous with S2 and further being capable of being extended along said analyte.

The invention also includes kits comprising in packaged combination (a) a polynucleotide primer having a 3'-terminus comprised of a nucleotide monophosphate containing at least one phosphorous-sulfur bond, (b) nucleoside triphosphates, and (c) a template-dependent polynucleotide polymerase. A kit for amplification of a target polynucleotide sequence can also comprise a second polynucleotide primer. The primers are related in that a product of the extension of one along the target sequence serves as a template for the extension of the other. In the above kit the second primer can have a 3'-terminus comprised of a nucleotide monophosophate that contains at least one phosphorous-sulfur bond.

### Brief Description of the Drawings

Figs. 1-3 are schematics of different embodiments in accordance with the present invention.

Figs. 4-6 are photographs of agarose gels of reactions products obtained in the EXAMPLES section hereinbelow.

In the attached Figs., these are referred to as the Figs. of the second aspect of the invention.

### Description of the Specific Embodiments

In its broadest aspect the present invention provides for the formation of a polynucleotide sequence complementary to a single stranded target polynucleotide sequence. In the method a polynucleotide primer having a 3'-terminus comprised of a nucleotide monophosphate in which at least one phosphate oxygen is replaced by sulfur is hybridized to the 3'-end of a target polynucleotide sequence. The polynucleotide primer is extended along the target polynucleotide sequence and extended polynucleotide primer is dissociated from the target polynucleotide sequence.

Heretofore, it had been thought that oligonucleotides containing thiophosphate groups were poor substrates for polymerases. See, for example, Gao, et al., supra. As a result of the present invention synthetic oligonucleotide primers that are resistant to degradation by the exonuclease activities of many DNA polymerases can be used in amplification procedures. Accordingly, polymerases that have 3' to 5' exonuclease activity can be employed in various extension and amplification procedures. The editing function provided by this exonuclease activity allows for greater fidelity of replication in an amplification procedure.

The methods have particular application in the area of the amplification of polynucleotides such as by polymerase chain reaction, single primer amplification and amplification using oligonucleotides of random sequence as primers, all referred to above, in which a polynucleotide sequence derived from a sample is amplified. The present method provides a highly convenient method for conducting amplification of nucleic acid sequences.

As a result of the present invention synthetic oligonucleotide primers can be used in extension procedures including nucleic acid amplification without concern for degradation by the exonuclease activities associated with many DNA polymerases used in nucleic acid extension and amplification procedures. For example, PCR, which provides for an exponential amplification of nucleic acids, can be carried out using an enzyme other than a Taq polymerase, which lacks a 3'-5' exonuclease activity and, thus, is not capable of removing mismatches. Numerous modifications of this procedure have been described, some requiring temperature cycling, others amplifying at a single temperature. The present invention allows amplification procedures such as PCR to be carried out using a single enzyme. Another advantage of the present invention is in the area of detection of genetic polymorphisms or point mutations in diagnosing genetic disease routinely described using PCR. Primers mismatched at the 3' end can be used to introduce artificial restriction sites or to detect single base mismatches in the target DNA together with enzymes having 3' exonuclease activities without concern that the enzyme would digest the mismatched primer-template region.

Before proceeding further with a description of the specific embodiments of the present invention, a number of terms will be defined. (All other terms are as defined above.)

Target polynucleotide sequence -- a sequence of nucleotides to be identified, usually existing within a polynucleotide analyte, the identity of which is known to an extent sufficient to allow preparation of various primers and other molecules necessary for conducting an amplification of the target sequence contained within. In general, the primers hybridize to, and are extended along, the target polynucleotide sequence and, thus, the target polynucleotide sequence acts as a template. The target polynucleotide sequence usually lies between two defined sequences but need not. In general, the primers and other probe polydeoxynucleotides hybridize with the defined sequences or with at least a portion of such target sequence, usually at least a ten nucleotide segment at the 3'-end thereof and preferably at least 15, frequently 20 to 50 nucleotide segment thereof. As mentioned above, the target polynucleotide sequence generally has two defined, that is, non-contiguous, complementary or non-complementary, nucleotide sequences, S1 and S2, which are capable of hybridizing to such primers or probes. The target polynucleotide sequence usually contains from about 30 to 5,000 or more nucleotides, preferably 50 to 1,000 nucleotides. The two non-contiguous, non-complementary nucleotide sequences, S1 and S2, preferably contain from 10 to 100 nucleotides each and are separated by at least ten bases, preferably at least 100, usually 200 to 10,000. The target polynucleotide sequence is frequently a part of the polynucleotide analyte. The target polynucleotide sequence is generally a fraction of a larger molecule or it may be substantially the entire molecule. The minimum number of nucleotides in the target polynucleotide sequence is selected to assure that the presence of target polynucleotide sequence in a sample is a specific indicator of the presence of polynucleotide analyte in a sample. Very roughly, the sequence length is usually greater than about 1.6 log L nucleotides where L is the number of base pairs in the genome of the biologic source of the sample. The maximum number of nucleotides in the target sequence is normally governed by the length of the polynucleotide analyte and its tendency to be broken by shearing, or other processes during isolation and any procedures required to prepare the sample for assay and the efficiency of detection and/or amplification of the sequence.

Polydeoxynucleotide primer -- a polydeoxynucleotide, usually a synthetic deoxynucleotide that is single stranded, containing a sequence at its 3'-end that is capable of hybridizing with a defined sequence of the target polynucleotide sequence. Normally a polydeoxynucleotide primer has at least 50%, preferably 70%, more preferably 90%, most preferably 100%, complementarity as the defined sequence. The number of deoxynucleotides in the hybridizable sequence of a polydeoxynucleotide primer should be such that stringency conditions used to hybridize the polydeoxynucleotide primer will prevent excessive random non-specific hybridization. Usually, the number of deoxynucleotides in the polydeoxynucleotide primer will be at least as great as the defined sequence of the target polynucleotide sequence, namely, at least ten deoxynucleotides, preferably at least 15 deoxynucleotides and generally from about 10 to 200, preferably 20 to 50, deoxynucleotides.

Means for extending a primer -- a polynucleotide polymerase or a single stranded template polynucleotide having a sequence other than at its 3' end that can hybridize to at least the 3' end of the primer or both. Means for extending a primer also includes nucleoside triphosphates or analogs thereof capable of acting as substrates for the enzyme and other materials and conditions required for enzyme activity such as a divalent metal ion (usually magnesium), pH, ionic strength, organic solvent (such as formamide), and the like.

In accordance with the present invention at least one polydeoxynucleotide primer used in an extension along a template or target polynucleotide has a 3'-terminus comprised of a nucleotide monophosphate in which an oxygen of at least one phosphate has been replaced by sulfur. Preferably, an oxygen of 1 to 5 phosphates is replaced by sulfur, more preferably, the oxygen of 1 to 2 phosphates is replaced. The sulfur is frequently bound solely to phosphorus (phosphorothioate group), but can also be bound to a ribose carbon atom or carbon atom of a label. Thus, the polydeoxynucleotide primers utilizable in the methods of the present invention contain at least one, preferably 1 to 5, more preferably, 1 to 2, phosphorus-sulfur bonds. One consideration in the number of phosphorus-sulfur bonds is the degree of complementarity between the primer and the template polynucleotide. In general, the greater the complementarity, the greater the permissible number of phosphorus-sulfur bonds. The phosphorus-sulfur bonds are generally located at the 3' end of the polydeoxynucleotide primer, usually within the last ten nucleotides from the 3' end, preferably within the last five nucleotides from the 3' end, more preferably, at the last nucleotide of the primer. These sulfur-containing polydeoxynucleotide primers can be prepared according to known techniques as described below.

In one embodiment of the present invention a method is described for forming a polynucleotide sequence complementary to a single stranded target polynucleotide sequence ("target sequence"). The method comprises the steps of (a) hybridizing to the 3'-end of the target sequence a polynucleotide primer having a 3'-terminus comprised of a nucleotide monophosphate in which at least one phosphate oxygen is replaced by sulfur, (b) extending the polynucleotide primer along the target sequence and (c) dissociating the extended polynucleotide primer from the target sequence.

Such an embodiment of the method is depicted schematically in Fig. 1. Primer P1 has a phosphorus-sulfur bond (indicated by -S in the Fig.) located at its 3'-end. The primer hybridizes with T1 of the target polynucleotide sequence (T). The primer is extended along T to produce an extended primer H, which is complementary to T. T can be chosen such that a particular sequence, such as, for example, T2, is known. The extended primer contains sequence P2, which is complementary to T2. Next, the extended primer is dissociated from its complex.

Another embodiment of the present invention is a method for forming a polynucleotide sequence having a sequence identical, except for the presence of a phosphorus-sulfur bond in place of a phosphorus-oxygen bond, to a single stranded target polynucleotide sequence. The method comprises the steps of (a) hybridizing to the 3'-end of said target polynucleotide sequence a first polynucleotide primer ("first primer") containing at least one phosphorous-sulfur bond (b) extending the first primer along the target polynucleotide sequence (c) dissociating the extended first primer from the target polynucleotide (d) hybridizing to the 3'-end of the extended first primer a second polynucleotide primer ("second primer") wherein the second primer can be the same as or different from the first primer, and (e) extending the second primer along the extended first primer to form extended second primer wherein, when the first and second primers are the same, the extended first primer is a template for the first primer and, when the first and second primers are different, the extended first primer is a template for the second primer and the extended second primer is a template for the first primer. The above method can include the step of dissociating the extended second primer from the extended first primer. Conditions can be chosen to obtain repeated cycling such that extended primers are dissociated from their templates and then the primers can be hybridized to and extended along the extended primers. Cycling can be repeated at least 3 times. The extending is carried out in the presence of nucleoside triphosphates and template dependent polynucleotide polymerase.

The methods of the invention find use in polynucleotide amplification such as, for example, PCR and single primer amplification, wherein one or more copies of a target polynucleotide sequence, i.e., sequences identical to the target polynucleotide sequence, are formed.

The use of the present method in single primer amplification is depicted in Fig. 2. In the following description appropriate conditions for the hybridizing, extending, and dissociating steps are chosen as discussed below. Polydeoxynucleotide primer P1 hybridizes with T1, wherein T1 is capable of hybridizing with, preferably complementary to, T2 of the target polynucleotide sequence. P1 can also comprise a label W (not shown). P1 is hybridized with and extended along the target T to form extended primer H comprising sequences P1 and P2, wherein P2 is complementary to T2 and P1. H is dissociated from T and P1 hybridizes with P2 of H and T1 of T and is extended along H and T, respectively. The duplexes are dissociated and P1 is hybridized with and extended along H and T to yield H' and H, respectively. Further repetition results in multiple copies of the target polynucleotide sequence, which can be detected because of the presence of label W.

Another embodiment of this aspect of the present invention is depicted in Fig. 3 wherein primers each having a nucleotide monophosphate containing at least one phosphorus-sulfur bond are employed in a PCR amplification. It is, of course, within the purview of the present invention to conduct PCR with two primers, only one of which has a nucleotide monophosphate containing at least one phosphorus-sulfur bond. As mentioned above, the PCR method can be used for forming multiple copies of at least one double stranded polynucleotide sequence. The sequence comprises a single stranded target polynucleotide sequence and its complementary sequence. A sample suspected of containing one or more of the double stranded polynucleotide sequences is treated with polynucleotide primers P'1 and P'2, each of which, respectively, is capable of hybridizing to the target and its complementary sequence suspected of being present in the sample. Conditions are chosen for hybridizing the primers to, and extending the primers along, the target and the complementary sequences. The primers are selected such that the extension product formed from one primer, when it is dissociated from its complement, can serve as a template for the formation of the extension product of the other primer. As can be seen in Fig. 3, the complementary sequences are T' and T''. T' has sequences T'1 and T'2 and T'' has sequences T''1 and T''2. P'1 hybridizes with T'1 of T' and P'2 hybridizes with T''2 of T''. Under appropriate conditions P'1 is extended along T' to give V and P'2 is extended along T'' to give V'. It is apparent from Fig. 3 that P'1 and T'1 are complementary, P'2 and T''2 are complementary, P'1 and T''1 are homologous and P'2 and T'2 are homologous. The extended primers are dissociated from their respective templates. Molecules of primer P'1 hybridize to and are extended along T' and V', respectively, and molecules of primer P'2 hybridize to and are extended along T'' and V, respectively, to yield V, W', W and V', respectively. As can be seen from Fig. 3, repetition of the cycles results in multiple copies of W and W' containing the target sequences. The cycles are carried out under conditions such that a primer extension product is formed using the single strands produced as templates, resulting in amplification of the target sequences and complementary sequences if present.

The method has application where the target polynucleotide sequence is DNA or RNA. In one aspect the polydeoxynucleotide primer is labeled with a label (reporter molecule). The reporter molecule can be, for example, a detectable group or a binder such as biotin or a nucleotide sequence other than the sequence that hybridizes with the target sequences. The reporter molecule may be attached directly to a phosphorothioate group in the primer or may be attached elsewhere. The extended primer(s) can be detected by means of a reporter molecule covalently bonded to a probe. The probe will usually have a nucleotide sequence that is homologous or complementary to a portion of the target nucleotide sequence other than those sequences to which the primers bind.

Another embodiment of the invention concerns a method for detecting the presence of a polynucleotide analyte in a sample suspected of containing the polynucleotide analyte. A medium containing the sample is treated as described above to form a single stranded target polynucleotide sequence from the polynucleotide analyte, if present. The target polynucleotide sequence has two non-contiguous, non-complementary nucleotide sequences S1 and S2 wherein S2 is 5' of S1, and is at least ten nucleotides long. The medium is combined with an extender probe having two deoxynucleotide sequences. Such an approach to single primer amplification is described above. The sequence at the 3'-end of the extender probe (EP1) is hybridizable with S1. The other of the deoxynucleotide sequences (EP2) is homologous to S2. A polydeoxynucleotide primer capable of hybridizing with a nucleotide sequence complementary to S2 is included when modification of the extender probe does not provide a primer. The extender probe is present in a concentration that is much less than the concentration of the primer, usually one percent or less. In accordance with the present invention the primer has a 3' terminus comprised of a nucleotide monophosphate in which at least one phosphate oxygen is replaced by sulfur. Deoxynucleoside triphosphates and one or more polydeoxynucleotide polymerases are also combined. Conditions are chosen such that (1) the extender probe is hybridized with and is extended along the target polynucleotide sequence to form a duplex, (2) the extender probe not hybridized with the target polynucleotide sequence is modified, (3) the extended extender probe is dissociated from the duplex, (4) the primer hybridizes with and is extended along the extended sequence to form a second duplex comprising extended primer, (5) the extended primer is dissociated from the duplex, and (6) the primer hybridizes with and is extended along said extended primer to form a duplex comprising extended primer. Steps (5) and (6) are repeated and steps (a) and (b) are performed concomitantly or wholly or partially sequentially. Then, an examination is conducted for the presence of the extended primer, the presence thereof indicating the presence of the polynucleotide analyte. Steps (5) and (6) are repeated a least three times, preferably, at least 10 times; usually it is preferable that the number of repetitions be less than 30. Generally, steps (5) and (6) are repeated a number of times sufficient to provide an accurate detection of the polynucleotide analyte. Where the polynucleotide analyte is RNA, the polydeoxynucleotide polymerase comprises a reverse transcriptase.

The conditions, concentrations etc. for carrying out these methods are analogous to those described above, unless noted below.

Where the present method is utilized in single primer amplification or in PCR, the method is conducted for a time sufficient to achieve a desired number of copies of the extended primer or a sequence complementary thereto. This, in turn, depends on the purpose for which the amplification is conducted, such as, for example, an assay for a polynucleotide analyte. Generally, the time period for conducting the method will be from about 1 to 10 minutes per cycle and any number of cycles can be used from 1 to as high as 200 or more, usually 5 to 80, frequently 10-60. As a matter of convenience it is usually desirable to minimize the time period and the number of cycles. In general, the time period for a given degree of amplification can be shortened, for example, by selecting concentrations of nucleoside triphosphates sufficient to saturate the polynucleotide polymerase and by increasing the concentrations of polynucleotide polymerase and polynucleotide primer. Generally, the time period for conducting the method will be from about 5 to 200 minutes. As a matter of convenience, it will usually be desirable to minimize the time period.

As mentioned above, a primary advantage of the present invention is that a template dependent polynucleotide polymerase having 3' exonuclease activity can be used in amplification of polynucleotides. The concentration of this polymerase will be chosen to be sufficient to accomplish chain extension. The concentration of the template-dependent polynucleotide polymerase is usually determined empirically. Preferably, a concentration is used that is sufficient such that further increase in the concentration does not decrease the time for the amplification by over 5-fold, preferably 2-fold. The primary limiting factor generally is the cost of the reagent.

The copies of extended primer(s) can be detected in numerous ways. For example, in the present method, molecules of the polydeoxynucleotide primer can be labeled with a reporter molecule such as a ligand, a small organic molecule, a polynucleotide sequence, a protein, support, a member of an operator-repressor pair, intercalation dye and the like. Any standard method for specifically detecting nucleic acid sequences can be used. It is important to note that, since sulfur atoms are being incorporated into the products of the amplification in accordance with the present invention, these sulfur atoms can constitute a reporter molecule, the presence of which is related to the presence of the target polynucleotide. In this approach the products of the amplification can be treated in a number of ways chemically to detect the presence of sulfur atoms. For example, the products of the reactions in accordance with the present invention can be treated with an enzyme that cleaves the bond opposite the phosphorothioate linkage in the double stranded product. Such an enzyme is, for instance, Hinc II. In another approach the products of the reactions can be treated chemically by known procedures to cleave the phosphorothioate bond. Other ways of detecting the presence of sulfur atoms in the reaction products will be suggested to those skilled in the art.

One method for detecting nucleic acids is to employ nucleic acid probes. One method utilizing probes is described in U.S. Patent Application Serial No. 773,386, filed September 6, 1985, US4868104.

Other assay formats and detection formats are disclosed in U.S. Patent Applications Serial Nos. 07/299,282 and 07/399,795 filed January 19, 1989, and August 29, 1989, respectively, see also EP379369, and U.S. Patent Application Serial No. 07/555,323 filed July 19, 1990, see also EP469755.

Examples of particular labels or reporter molecules and their detection are described above.

Detection of the signal is described above.

Various techniques can be employed for preparing a polydeoxynucleotide primer, or other polynucleotide sequences, as described above.

Polydeoxynucleotide primers containing at least one monophosphate having a 3' terminus comprised of a nucleotide monophosphate in which at least one phosphate oxygen is replaced by sulfur can be prepared according to known techniques. By way of illustration, see as described above in relation to extender probes containing such groups.

As a matter of convenience, predetermined amounts of reagents employed in the present invention can be provided in a kit in packaged combination. A kit can comprise in packaged combination (a) a polynucleotide primer having a 3'-terminus comprised of a nucleotide monophosphate containing at least one phosphorous-sulfur bond, (b) nucleoside triphosphates, and (c) a template-dependent polynucleotide polymerase, which may or may not have exonuclease activity. A kit for amplification of a target polynucleotide sequence comprises the above items and for conducting PCR would include in addition a second polynucleotide primer, where the primers are related in that a product of the extension of one along said target sequence serves as a template for the extension of the other. The second primer can also have a 3'-terminus comprised of a nucleotide monophosophate that contains at least one phosphorous-sulfur bond.

In assaying for a polynucleotide analyte in a sample, a kit useful in the present method can comprise, in packaged combination with other reagents mentioned above, reagents for forming a target polynucleotide sequence from a polynucleotide analyte. Furthermore, the polydeoxynucleotide primer can be labeled or can be provided with groups to render the sequence labeled or bound to a support. The kit can further include a labeled polynucleotide probe capable of binding to an amplified target polynucleotide sequence. The kits above can further include in the packaged combination deoxynucleoside triphosphates such as deoxynucleoside triphosphates, e.g., deoxyadenosine triphosphate (dATP), deoxyguanosine triphosphate (dGTP), deoxycytidine triphosphate (dCTP) and deoxythymidine triphosphate (dTTP). The kit can further include members of a signal producing system and also various buffered media, some of which may contain one or more of the above reagents.

The relative amounts of the various reagents in the kits can be varied widely to provide for concentrations of the reagents which substantially optimize the reactions that need to occur during the present method and to further substantially optimize the sensitivity of the assay. Under appropriate circumstances one or more of the reagents in the kit can be provided as a dry powder, usually lyophilized, including excipients, which on dissolution will provide for a reagent solution having the appropriate concentrations for performing a method or assay in accordance with the present invention. Each reagent can be packaged in separate containers or some reagents can be combined in one container where cross-reactivity and shelf life permit.

### EXAMPLES

The second aspect of this invention is demonstrated further by the following illustrative examples. Temperatures are in degrees centigrade (°C) and parts and percentages are by weight, unless otherwise indicated.

### EXAMPLE 1

### Preparation of 415mer Having an Inverted Repeat

The preparation of a 415mer having an inverted repeat (stem-loop molecule) was carried out in accordance with the method described in U.S. Patent Application Ser. No. 07/555,323 filed July 19, 1990, see also EP469755. In the preparation 10 attomoles of single-stranded M13mp19 (target DNA, Bethesda Research Laboratories (BRL)) in a 100 microliter reaction containing 10mM Tris-Cl, pH 8.3, 50mM KCl, 1.5mM MgCl₂, 0.01% gelatin and 200 micromolar deoxynucleotide triphosphates (dNTPs) was used. In the presence of 1 micromolar single primer amplification primer ON1 (polydeoxynucleotide primer) and 0.1 micromolar extender probe ON2, the above mixture was heated to 95°C for 5 minutes and cooled to room temperature for 15 minutes. This allowed the extender probe to anneal to target DNA. Taq polymerase (Stratagene, Inc., San Diego, CA) was added (5 units) and the temperature was cycled as follows: 90°C - 30 seconds, 55°C - 60 seconds, 72°C - 90 seconds. This temperature profile was repeated 30 times. The 415mer product was visualized by agarose gel electrophoresis. The agarose gels were prepared with SeaKem LE ararose (FMC BioProducts, Rockland, ME) 1.2% weight/volume (w/v)). The reaction mixture (10 microliters) was added to a 6X dye (0.25% bromophebol blue, 0.25% xylene cyanol, and 15% Ficoll, type 400 (Maniatis, et al., "Molecular Cloning, A Laboratory Manual", 455 (1982)) in water and subjected to electrophoreses in 1X TBE buffer (0.1M Tris base, 89 mM boric acid, and 2mM sodium ethylenediaminetetraacetate). The band constituting the 415mer product was identified by ethidium bromide (1mg/ml) staining and was detected by examination under UV light. Gels were photographed using a Polaroid MP-4 Land camera equipped with a UV transilluminator from Fotodyne, Inc., New Berlin, WI. The reaction mixtures that gave a single band corresponding to the 415mer were pooled and ethanol precipated according to standard procedures ("Current Protocols in Molecular Biology," Chapter 15, page 15.2.2) and quantified using a fluorometer.

Oligonucleotides ON1 and ON2 were as follows:

ON1:  5' TGT TGT TCC GTT AGT TCG TTT TAT T 3' (SEQ ID
NO: 2)

ON2: 5' TGT TGT TCC GTT AGT TCG TTT TAT TCA TAG TTA GCG
TAA CGA TCT AAA GTT TTG TC 3' (SEQ ID NO:7)

### EXAMPLE 2

### Amplification Using Primers with Phosphorothioate(s) at the 3' Pentultimate Position (s)

The 415mer produced as described in EXAMPLE 1 was utilized as a target sequence in single primer amplification reactions using several different primers containing one or more phosphorus-sulfur bonds in place of phosphorus-oxygen bonds as described more fully below. Primer 1 was ON1 with a phosphorus-sulfur bond at the 3' end between the ultimate and penultimate nucleotides as indicated in bold face as follows: 5' TGT TGT TCC GTT AGT TCG TTT TA**T T** 3'(SEQ ID NO:8). Primer 1 was prepared as described below for Primer 2.

Primer 2 was prepared by exonuclease digestion of the following oligonucleotide (ON3): 5' TGT TGT TCC GTT AGT TCG TTT **TAT T**CA TAG TTA GCG TAA CGA TCT AAA GTT TTG TC 3' (SEQ ID NO:9), which was synthesized in an automated (4-column Biosearch 8750 DNA synthesizer from Millipore Corp., Bedford, MA) manner until positioning of the thio-modified linkage(s), indicated in bold. Manual oxidations were then performed with 0.1M tetraethylthiuram disulfide (TETD) (Applied Biosystems, Inc., Foster City, CA) in acetonitrile. The remaining bases were added under normal coupling conditions following the protocol in Applied Biosystems, Inc., User Bulletin, Number 58, February 1991. The digestion of the above oligonucleotide (2µM) was carried out in 1X Vent buffer. The mixture was heat denatured for 5 minutes at 95°C and then cooled to room temperature. T7 DNA polymerase, unmodified Form II (New England Biolabs, Beverly, MA) was added and the reaction mixture was incubated for 5 minutes at 37°C. The T7 was then inactivated by heating for 2 minutes at 95°C. The resulting reaction mixture containing Primer 2 was used in the experiment below. Primer 2: 5' TGT TGT TCC GTT AGT TCG TTT **TAT T** 3' (SEQ ID NO:10).

The amplification reactions were carried out according to the following protocol: The 415mer was serially diluted in water to obtain a medium containing approximately 500 molecules of the 415mer. Vent polymerase buffer (1X) (100mM KCL, 200mM Tris-HCl, pH 8.8 at 25°C, 100mM [NH₄]₂SO₄, 20mM MgSO₄, 1% Triton® X-100), 300µM dNTPs, and 1µM of Primer 1 were combined. The combination was heated at 95°C for 5 minutes and then cooled at room temperature for 15 minutes. Taq DNA polymerase (Stratagene, San Diego, CA) (5 units) was added to the reaction mixture, which was subjected to temperature cycling for 50 cycles as follows: 72°C for 5 min (1X), 90°C for 30 sec, 55 C for 60 sec, and 72°C for (90 sec (50X). As a control the above amplification was repeated with a control primer, which is ON1 without the phosphorus-sulfur bonds in place of phosphorus-oxygen bonds, namely, 5' TGT TGT TCC GTT AGT TCG TTT TAT T 3' (SEQ ID NO:2). The results of the above experiments were determined by agarose gel electrophoreses as described above and are shown in Fig. 4, wherein the control reaction is shown in Lane 1 and the reactions using Primer 1 is shown in Lanes 2 and 3.

Amplification of the 415mer was also carried out using Primer 2 and a protocol similar to that described above. The concentration of Primer 2 was 1µM and 2.5 units of Pfu DNA polymerase (Stratagene, San Diego, CA) were used in place of the Tag DNA polymerase. The results are shown in Fig. 5. Lane 1 represents the negative control where no target molecules were present. Lanes 2, 3 and 4 represent amplification in accordance with the present invention of approximately 300, 30 and 3 of the 415mer molecules, respectively, where serial dilution was used to obtain the pre-amplification 415mer concentrations.

### EXAMPLE 3

### Single Primer Amplification Using a Degradable Phosphorothioate-containing Extender Probe Generating Primer In Situ

The detection of approximately 600 double-stranded target molecules using single primer amplification was demonstrated repeatedly using a degradable, phosphorothioate-containing oligonucleotide. The oligonucleotide (56 bases) acts as the extender probe in creating an amplifiable stem-loop and, following nuclease treatment, serves as a primer to drive amplification.

The synthesis of the extender probe oligonucleotide was carried out in an automated (4-column Biosearch 8750 DNA synthesizer) manner until positioning of the thio-modified linkage(s). Manual oxidations were then performed with 0.1M tetraethylthiuram disulfide (TETD) (Applied Biosystems, Inc., Foster City, CA) in acetonitrile. The remaining bases were added under normal coupling conditions following the protocol in Applied Biosystems, Inc., User Bulletin, Number 58, February 1991.

Extender Probe containing thio linkage(s): Three phosphorothioate linkages (arrows) between T₃₃ and T₃₆:
mixture of 3 primers (24, 23, 22 bases) remained after degradation (underlined)

3' TC ATT GTA CCT CGT CCA GCG CCT AAA GCT G T T T↓A↓T↓T
T T G C T T G A T TGCCTTGTTGT 5' (SEQ ID NO:6)

The formation and amplification of a stem-loop molecule was carried out in 100 microliter reactions containing an appropriate buffer (20 mM Tris-HCl, pH 8.8, 10 mM KC1, 10 mM (NH₄)₂SO₄, 2 mM MgSO₄, and 0.1% Triton X-100), dNTPs (200 to 300 micromolar), double stranded target polynucleotide molecule (600 M13mp19 molecules,), and the extender probe (0.5 and 1 micromolar initial concentration). Reactants were heat-denatured for 5 minutes at 95°C and annealed at 25°C (room temperature) for 15 to 20 minutes. Each of three DNA polymerases with strong 3' exonuclease activity were separately added (6 units per 100 µl of Klenow DNA polymerase, 5 units per 100 µl of T4 DNA polymerase, New England Biolabs (NEB), Beverly Mass or 5 units per 100 µl of T7 DNA polymerase, New England Biolabs (NEB), Beverly Mass.). Reactions were incubated at 37°C such that any extender probe annealed to target was chain extended, whereas all non-annealed extender probe was degraded up to the position(s) of the thio linkage(s). The extender probe was radiolabelled at the 5'-end to monitor degradation. Complete degradation of the non-annealed extender probe up to the sulfur linkage(s) was obtained in as little as 1 minute, while the remaining sequence was resistant to further degradation for up to 60 minutes. After the elongation/degradation was complete, reactants were again heated at 95°C for 2 minutes, thereby inactivating the particular DNA polymerase and denaturing the newly formed stem-loop molecule from the original target molecule. A heat-stable polymerase was added (Pfu from Stratagene San Diego, CA, 10 units per 100 µl) and the reactions are cycled as follows: 72°C for 5 min (1X), 90°C for 30 sec, 55°C for 60 sec, 72°C for 5 min (10X), 90°C for 30 sec, 55 C for 60 sec, and 72°C for (90 sec (50X). Aliquots from these reactions are analyzed by electrophoresis through 1.2% agarose (Seakem, FMC Bio Products) gels in 1 x TBE buffers [89 mM Tris-borate, 89 mM boric acid, 0.2 mM EDTA] and the amplified product was visualized by ethidium bromide staining. The results are shown in Fig. 6 where Lane 1 represents 0.5 micromolar concentration of the extender probe and Lane 2 represents 1 micromolar concentration of the extender probe.

### EXAMPLE 4

### PCR Amplification Using Phosphorothioate Primer

PCR amplification is carried out using 10 attomoles of single-stranded M13mp19 (target DNA, Bethesda Research Laboratories (BRL)) in a 100 microliter reaction containing 10mM Tris-Cl, pH 8.3, 50mM KCl, 1.5mM MgCl₂, 0.01% gelatin and 200 micromolar deoxynucleotide triphosphates (dNTPs). In the presence of 1.0 micromolar concentrations of Primer 3 (5'-GTT GAA ATT AAA CCA TCT CAA GCC C-3' (SEQ ID NO:11)) and Primer 4 (5'-CAT AGT TAG CGT AAC GAT CTA AAG TTT TG**T C**-3' (SEQ ID NO:12)), the above mixture is heated to 95°C for 5 minutes and cooled to room temperature for 15 minutes. Taq DNA polymerase (Stratagene) is added (5 units) and the temperature is cycled as follows: 90°C - 30 seconds, 55°C - 60 seconds, 72°C - 90 seconds. This temperature profile is repeated 30 times. The expected size of amplification product is 880 base pairs. Aliquots (5 microliters) taken after 0, 10, 20, and 30 cycles from the PCR reaction mixtures are electrophoresed on a 1% agarose gel and stained with ethidium bromide. Based on the mobilities of molecular weight standards, a single band of approximately 880 base pairs appears following 10, 20 and 30 cycles of PCR.

Target concentration is decreased to 10 attomoles/ 100 microliter reaction and amplified using PCR. Individual reaction components, as well as time and temperature cycling parameters, were as described previously. Reactions are heated to 95°C for 5 minutes and cooled to room temperture for 30 minutes. Aliquots (5 microliters) taken after 0, 15 and 30 cycles are electophoresed on a 0.8% agarose gel and stained as descirbed. A single band of approximately 880 base pairs appears following 30 cycles of PCR.

The above examples demonstrate that primers containing a 3' terminus comprised of a nucleotide monophosphate, which has at least one phosphate-sulfur bond in place of a phosphorus-oxygen bond can be utilized effectively in amplification of nucleic acids.

The above discussion includes certain theories as to mechanisms involved in the present invention. These theories should not be construed to limit the present invention in any way, since it has been demonstrated that the present invention achieves the results described.

The above description and examples fully disclose the invention including preferred embodiments thereof.

## Claims

1. A method for forming, from an extender probe and a single stranded target polynucleotide sequence, a polynucleotide free of unmodified extender probe, said polynucleotide having a sequence identical to said target polynucleotide sequence attached at its 3'-end to a polynucleotide sequence complementary to a polynucleotide sequence at the 5' end of said target polynucleotide sequence, said method comprising:
(a) hybridizing to the 3'-end of said single stranded target polynucleotide sequence the 3'-end of said extender probe wherein said extender probe contains a sequence substantially identical to a sequence S2 at the 5'-end of said single stranded target polynucleotide sequence,
(b) extending said extender probe along said single stranded target polynucleotide sequence,
(c) modifying said 3'-end of said extender probe not hybridized to said single stranded target polynucleotide sequence,
(d) hydridizing a primer to the 3'-end of the extended extender probe, said primer having said sequence S2 at its 3-end, and
(e) extending said primer along said extended extender probe.

2. The method of Claim 1 wherein said modifying comprises means for extending or degrading said 3'-end of said extender probe not hybridized to said single stranded target polynucleotide sequence.

3. A method for producing from an extender probe a polydeoxynucleotide (PDN) having two segments that are non-contiguous and complementary with each other, said PDN being substantially free of said extender probe, said method comprising the steps of:
providing in combination (a) a polynucleotide having two non-contiguous, non-complementary nucleotide sequences S1 and S2 wherein S2 is 5' of S1 and is at least ten nucleotides long, (b) an extender probe comprised of two deoxynucleotide sequences, wherein the sequence at the 3'-end of said extender probe, EP1, is hybridizable with S1 and the other of said deoxynucleotide sequences,EP2, is homologous to S2, and (c) means for chemically modifying the 3'-end of said extender probe that does not hybridize with said polynucleotide, and
extending said extender probe along said polynucleotide wherein the 3'-end of said extender probe not hybridized with said polynucleotide is modified.

4. The method of Claim 3 wherein said means comprises a nucleotide sequence EP3 capable of hybridizing with said EP1.

5. The method of Claim 3 wherein said means comprises a nucleotide sequence EP3 in said extender probe, said EP3 being capable of hybridizing with said EP1.

6. The method of Claim 3 wherein said means is means for degrading the 3'-end of said extender probe.

7. The method of Claim 6 wherein said means comprises an enzyme having 3'-exonuclease activity.

8. The method of Claim 6 wherein said extender probe is degraded to give a polydeoxynucleotide primer capable of hybridizing at least at its 3'-end with a nucleotide sequence complementary to S2.

9. The method of Claim 3 which further comprises providing in said combination a polydeoxynucleotide primer capable of hybridizing at least at its 3'-end with a nucleotide sequence complementary to S2 under conditions where (1) said extended extender probe is rendered single stranded, (2) said polydeoxynucleotide primer hybridizes with and is extended along said extended extender probe to form a duplex comprising extended primer, (3) said extended primer is dissociated from said duplex, and (4) said primer hybridizes with and is extended along said extended primer to form a duplex comprising extended primer.

10. The method of Claim 9 wherein steps (3) and (4) are repeated.

11. A method according to Claim 3 for replicating a target polynucleotide sequence, said target polynucleotide sequence having two non-contiguous, non-complementary nucleotide sequences S1 and S2 each at least 10 nucleotides long, wherein S2 is 5' of S1, said method comprising the step of:
providing in combination, either concomitantly or wholly or partially sequentially, (1) said target polynucleotide sequence, (2) an extender probe having two deoxynucleotide sequences wherein the sequence at the 3'-end of said extender probe, EP1, is hybridizable with S1 and the other of said deoxynucleotide sequences, EP2, is homologous to S2, (3) means for extending or degrading the 3'-end of said extender probe not hybridized with said target polynucleotide sequence, (4) a polydeoxynucleotide primer comprised of sequence S2 at its 3'-end where said primer may be provided directly or generated in situ, (5) DNA polymerase and (6) deoxynucleoside triphosphates under conditions wherein (A) some of said extender probe becomes hybridized with and extended (extended EP) along said target polynucleotide sequence to form a duplex, (B) extender probe not hybridized to said target nucleotide sequence is extended or degraded at its 3'-end (C) said extended EP is dissociated from said duplex, (D) said primer hybridizes with and is extended along said extended EP to form a duplex comprising extended primer, (E) said extended primer is dissociated from said duplex, and (F) said primer hybridizes with and is extended along said extended primer to form a duplex comprising extended primer and steps (E) and (F) are repeated.

12. The method of Claim 11 wherein said polydeoxynucleotide primer is provided by degradation of said extender probe, and wherein the phosphate nearest the 3'-end of said primer is replaced by a phosphorothioate.

13. A method according to Claim 3 for detecting the presence of a polynucleotide analyte, said method comprising the steps of:
(a) treating a medium suspected of containing said polynucleotide analyte to form a single stranded target polynucleotide sequence from said polynucleotide analyte, if present, said target polynucleotide sequence having two non-contiguous, non-complementary nucleotide sequences S1 and S2 wherein S2 is 5' of S1, and is at least ten nucleotides long,
(b) combining said medium with (1) an extender probe having two deoxynucleotide sequences wherein the sequence at the 3'-end of said extender probe (EP1) is hybridizable with S1 and the other of said deoxynucleotide sequence (EP2) is substantially identical to S2, (2) a nucleotide sequence (NS) having a portion capable of hybridizing with EP1 wherein said NS may be a separate molecule or part of said extender probe, (3) a polydeoxynucleotide primer capable of hybridizing with a nucleotide sequence complementary to S2, (4) deoxynucleoside triphosphates, (5) and DNA template dependent polydeoxynucleotide polymerase under conditions wherein (A) said extender probe is hybridized with and is extended (extended extender probe) along said target polynucleotide sequence to form a duplex, (B) said extender probe not hybridized with said target polynucleotide sequence is extended along NS, (C) said extended extender probe is dissociated from said duplex, (D) said primer hybridizes with and is extended along said extended extender probe to form a duplex comprising extended primer, (E) said extended primer is dissociated from said duplex, and (F) said primer hybridizes with and is extended along said extended primer to form a duplex comprising extended primer and steps (E) and (F) are repeated, wherein steps (a) and (b) are performed concomitantly or wholly or partially sequentially, and
(c) examining for the presence of said extended primer.

14. A method for forming a polynucleotide sequence identical to a single stranded target polynucleotide sequence said method comprising:
(a) combining in a medium said single stranded target polynucleotide sequence, one or more enzymes having DNA polymerase and 3'-exonuclease activity, and an extender probe comprised at its 3'end of a sequence EP1 complementary to a sequence S1 at the 3'-end of said single stranded target polynucleotide sequence and a sequence EP2 that is substantially identical to a sequence S2 at the 5'-end of said target sequence wherein said extender probe contains at least one phosphorothioate within said EP2 sequence, and
(b) treating said medium to cause hybridization of said extender probe to said single stranded target polynucleotide, extension of said extender probe along said single stranded target polynucleotide sequence, degradation of the 3'-end of said extender probe not hybridized to said single stranded target polynucleotide sequence to form a primer having at its 3'-end said EP2 sequence, hybridization of said primer to the extended extender probe, and extension of said primer along said extended extender probe.

15. A kit comprising in packaged combination:
an extender probe having at its 3'-end a sequence (EP1) hybridizable with a first sequence in a target polynucleotide sequence and having a sequence (EP2) that is substantially identical to a second sequence of said target polynucleotide sequence, wherein in said target polynucleotide sequence said second sequence is 5' and non-contiguous with said first sequence,
a nucleotide sequence (NS) having a portion capable of hybridizing with EP1 wherein said NS may be a separate molecule or part of said extender probe, and
a polydeoxynucleotide primer capable of hybridizing with a sequence that is complementary with said second sequence when means for forming said primer is not otherwise present.

16. A kit comprising an extender probe having at least one phosphorothioate diester and a 3'-exonuclease capable of hydrolyzing said extender probe.

17. A method according to Claim 3 for detecting the presence of a polynucleotide analyte, said method comprising the steps of:
(a) treating a medium suspected of containing said polynucleotide analyte to form a single stranded target polynucleotide sequence from said polynucleotide analyte, if present, said target polynucleotide sequence having two non-contiguous, non-complementary nucleotide sequences S1 and S2 wherein S2 is 5' of S1, and is at least ten nucleotides long,
(b) combining said medium with (1) an extender probe composed of two deoxynucleotide sequences wherein the sequence at the 3'-end of said extender probe (EP1) is hybridizable with S1 and the other of said deoxynucleotide sequences (EP2) is substantially identical to S2 wherein said extender probe contains at least one phosphorothioate diester and is capable of degrading to form a polydeoxynucleotide primer capable of hybridizing with a nucleotide sequence complementary to S2, (3) deoxynucleoside triphosphates, and (4) DNA template dependent polydeoxynucleotide polymerase under conditions wherein (A) said extender probe is hybridized with and is extended along said target polynucleotide sequence to form a duplex, (B) said extender probe not hybridized with said target nucleotide sequence is degraded to form said polydeoxynucleotide primer, (C) said extended extender probe is dissociated from said duplex, (D) said primer hybridizes with and is extended along said extended extender probe, (E) said extended primer is dissociated from said duplex, and (F) said primer hybridizes with and is extended along said extended primer to form a duplex comprising extended primer and steps (E) and (F) are repeated, wherein steps (a) and (b) are performed concomitantly or wholly or partially sequentially, and
(c) examining for the presence of said extended primer.

18. A method for forming a polynucleotide sequence complementary to a single stranded target polynucleotide sequence ("target sequence"), said method comprising the steps of:
(a) hybridizing to the 3'-end of said target sequence a polynucleotide primer having a 3'-terminus comprised of a nucleotide monophosphate in which a sulfur is in place of at least one phosphate oxygen,
(b) extending said polynucleotide primer along said target sequence and
(c) dissociating said extended polynucleotide primer from said target sequence.

19. The method of Claim 18 wherein a sulfur is in place of 1 to 5 phosphate oxygens.

20. A method according to Claim 18 for forming a polynucleotide sequence having a sequence identical, except for the presence of a phosphorus-sulfur bond in place of a phosphorus-oxygen bond, to a single stranded target polynucleotide sequence ("target sequence"), said method comprising the steps of:
(a) hybridizing to the 3'-end of said target sequence a first polynucleotide primer ("first primer") containing at least one phosphorous-sulfur bond,
(b) extending said first primer along said target sequence,
(c) dissociating said extended first primer from said target sequence,
(d) hybridizing to the 3'-end of said extended first primer a second polynucleotide primer ("second primer") wherein said second primer can be the same as or different from said first primer, and
(e) extending said second primer along said extended first primer to form extended second primer wherein, when said first and second primers are the same, said extended first primer is a template for said first primer and, when said first and second primers are different, said extended first primer is a template for said second primer and said extended second primer is a template for said first primer.

21. The method of Claim 20 wherein said first and second primers are the same.

22. The method of Claim 20 or 21 wherein conditions are chosen to obtain repeated cycling such that extended primers are dissociated from their templates and said primers are then hybridized to and extended along said extended primers.

## Patentansprüche

1. Verfahren zur Herstellung aus einer Verlängerungssonde und einer einzelsträngigen Target-Polynukleotidsequenz eines Polynukleotids, das frei von nicht modifizierter Verlängerungssonde ist, wobei das Polynukleotid eine Sequenz aufweist, die mit der Target-Polynukleotidsequenz identisch ist und an ihrem 3'-Ende mit einer Polynukleotidsequenz verknüpft ist, die komplementär zu einer Polynukleotidsequenz am 5'-Ende der Target-Polynukleotidsequenz ist, welches Verfahren umfaßt:
(a) Hybridisierung des 3'-Endes der einzelsträngigen Target-Polynukleotidsequenz mit dem 3'-Ende der Verlängerungssonde, wobei die Verlängerungssonde eine Sequenz enthält, die im wesentlichen mit einer Sequenz S2 am 5'-Ende der einzelsträngigen Target-Polynukleotidsequenz identisch ist,
(b) Verlängerung der Verlängerungssonde entlang der einzelsträngigen Target-Polynukleotidsequenz,
(c) Modifizierung des 3'-Endes der nicht mit der einzelsträngigen Target-Polynukleotidsequenz hybridisierten Verlängerungssonde,
(d) Hybridisierung eines Primers mit dem 3'-Ende der verlängerten Verlängerungssonde, welcher Primer die genannte Sequenz S2 an seinem 3'-Ende aufweist, und
(e) Verlängerung des Primers entlang der verlängerten Verlängerungssonde.

2. Verfahren nach Anspruch 1, worin die Modifizierung umfaßt Mittel zur Verlängerung oder zum Abbau des 3'-Endes der nicht mit der einzelsträngigen Target-Polynukleotidsequenz hybridisierten Verlängerungssonde.

3. Verfahren zur Herstellung aus einer Verlängerungssonde eines Polydesoxynukleotids (PDN) mit zwei Segmenten, die nicht aneinander angrenzen und komplementär zueinander sind, wobei das PDN im wesentlichen frei von der Verlängerungssonde ist, welches Verfahren umfaßt die Schritte:
der Bereitstellung in Kombination von (a) einem Polynukleotid mit zwei nicht aneinander angrenzenden, nicht-komplementären Nukleotidsequenzen S1 und S2, wobei S2 5' von S1 liegt und mindestens 10 Nukleotide lang ist, (b) einer Verlängerungssonde, die zwei Desoxynukleotidsequenzen umfaßt, wobei die Sequenz am 3'-Ende der Verlängerungssonde, EP1, mit S1 hybridisierbar ist und die andere Desoxynukleotidsequenz EP2 homolog zu S2 ist, und (c) einem Mittel zur chemischen Modifizierung des 3'-Endes der nicht mit dem Polynukleotid hybridisierenden Verlängerungssonde, und
der Verlängerung der Verlängerungssonde entlang des Polynukleotids, wobei das 3'-Ende der nicht mit dem Polynukleotid hybridisierten Verlängerungssonde modifiziert ist.

4. Verfahren nach Anspruch 3, worin das Mittel eine Nukleotidsequenz EP3 umfaßt, die zur Hybridisierung mit EP1 imstande ist.

5. Verfahren nach Anspruch 3, wobei das Mittel eine Nukleotidsequenz EP3 in der Verlängerungssonde umfaßt, wobei EP3 imstande ist, mit EP1 zu hybridisieren.

6. Verfahren nach Anspruch 3, worin das Mittel ein Mittel zum Abbau des 3'-Endes der Verlängerungssonde ist.

7. Verfahren nach Anspruch 6, worin das Mittel ein Enzym mit 3'-Exonuklease-Aktivität umfaßt.

8. Verfahren nach Anspruch 6, worin die Verlängerungssonde abgebaut wird, um einen Polydesoxynukleotid-Primer zu ergeben, der imstande ist, mindestens an seinem 3'-Ende mit einer zu S2 komplementären Nukleotidsequenz zu hybridisieren.

9. Verfahren nach Anspruch 3, welches ferner umfaßt die Bereitstellung in dieser Kombination von einem Polydesoxynukleotid-Primer, der imstande ist, mindestens an seinem 3'-Ende mit einer zu S2 komplementären Nukleotidsequenz zu hybridisieren, unter Bedingungen, unter denen (1) die verlängerte Verlängerungssonde einzelsträngig gemacht wird, (2) der Polydesoxynukleotid-Primer mit der verlängerten Verlängerungssonde hybridisiert und daran entlang verlängert wird, um einen Doppelstrang, der verlängerten Primer umfaßt, zu bilden, (3) der verlängerte Primer von dem Doppelstrang dissozilert wird, und (4) der Primer mit dem verlängerten Primer hybridisiert und daran entlang verlängert wird, um einen Doppelstrang zu bilden, der verlängerten Primer umfaßt.

10. Verfahren nach Anspruch 9, worin die Schritte (3) und (4) wiederholt werden.

11. Verfahren nach Anspruch 3 zur Replizierung einer Target-Polynukleotidsequenz, welche Target-Polynukleotidsequenz zwei nicht aneinander angrenzende, nicht-komplementäre Nukleotidsequenzen S1 und S2 umfaßt, die jeweils mindestens 10 Nukleotide lang sind, wobei S2 5' von S1 liegt, welches Verfahren umfaßt den Schritt:
der Bereitstellung in Kombination, entweder gleichzeitig oder ganz oder teilweise sequentiell, von (1) der Target-Polynukleotidsequenz, (2) einer Verlängerungssonde mit zwei Desoxynukleotidsequenzen, wobei die Sequenz am 3'-Ende der Verlängerungssonde, EP1, mit S1 hybridisierbar ist und die andere Desoxynukleotidsequenz EP2 homolog zu S2 ist, (3) einem Mittel zur Verlängerung oder zum Abbau des 3'-Endes der mit der Target-Polynukleotidsequenz nicht hybridisierten Verlängerungssonde, (4) einem Polydesoxynukleotid-Primer, der die Sequenz S2 an seinem 3'-Ende umfaßt, wobei der Primer direkt bereitgestellt oder in situ erzeugt werden kann, (5) DNA-Polyrnerase und (6) Desoxynukleosid-Triphosphaten unter Bedingungen, unter denen (A) ein Teil der Verlängerungssonde mit der Target-Polynukleotidsequenz hybridisiert und daran entlang verlängert wird (verlängerte EP), um einen Doppelstrang zu bilden, (B) nicht mit der Target-Nukleotidsequenz hybridisierte Verlängerungssonde an ihrem 3'-Ende verlängert oder abgebaut wird, (C) die verlängerte EP von dem Doppelstrang dissoziiert wird, (D) der Primer mit der verlängerten EP hybridisiert und daran entlang verlängert wird, um einen Doppelstrang zu bilden, der verlängerten Primer umfaßt, (E) der verlängerte Primer von dem Doppelstrang dissoziiert wird, und (F) der Primer mit dem verlängerten Primer hybridisiert und daran entlang verlängert wird, um einen Doppelstrang zu bilden, der verlängerten Primer umfaßt, und die Schritte (E) und (F) wiederholt werden.

12. Verfahren nach Anspruch 11, worin der Polydesoxynukleotid-Primer bereitgestellt wird durch Abbau der Verlängerungssonde und worin das dem 3'-Ende des Primers nächste Phosphat durch ein Phosphorothioat ersetzt wird.

13. Verfahren nach Anspruch 3 zum Nachweis der Anwesenheit eines Polynukleotid-Analyten, welches Verfahren umfaßt die Schritte:
(a) der Behandlung eines Mediums, von dem angenommen wird, daß es den Polynukleotid-Analyten enthält, um aus dem Polynukleotid-Analyten, falls vorhanden, eine einzelsträngige Target-Polynukleotidsequenz zu bilden, welche Target-Polynukleotidsequenz zwei nicht aneinander angrenzende, nicht-komplementäre Nukleotidsequenzen S1 und S2 aufweist, wobei S2 5' von S1 liegt und mindestens 10 Nukleotide lang ist,
(b) der Kombination des Mediums mit (1) einer Verlängerungssonde mit zwei Desoxynukleotidsequenzen, wobei die Sequenz am 3'-Ende der Verlängerungssonde (EP1) mit S1 hybridisierbar ist und die andere Desoxynukleotidsequenz (EP2) im wesentlichen mit S2 identisch ist, (2) einer Nukleotidsequenz (NS), die einen mit EP1 hybridisierbaren Abschnitt aufweist, wobei die NS ein separates Molekül oder Teil der Verlängerungssonde sein kann, (3) einem Polydesoxynukleotid-Primer, der imstande ist, mit einer zu S2 komplementären Nukleotidsequenz zu hybridisieren, (4) Desoxynukleosid-Triphosphaten, und (5) DNA-Matrizenabhängiger Polydesoxynukleotid-Polymerase unter Bedingungen, unter denen (A) die Verlängerungssonde mit der Target-Polynukleotidsequenz hybridisiert und daran entlang verlängert wird (verlängerte Verlängerungssonde), um einen Doppelstrang zu bilden, (B) die nicht mit der Target-Polynukleotidsequenz hybridisierte Verlängerungssonde entlang NS verlängert wird, (C) die verlängerte Verlängerungssonde von dem Doppelstrang dissoziiert wird, (D) der Primer mit der verlängerten Verlängerungssonde hybridisiert und daran entlang verlängert wird, um einen Doppelstrang zu bilden, der verlängerten Primer umfaßt, (E) der verlängerte Primer von dem Doppelstrang dissoziiert wird, und (F) der Primer mit dem verlängerten Primer hybridisiert und daran entlang verlängert wird, um einen Doppelstrang zu bilden, der verlängerten Primer umfaßt, und die Schritte (E) und (F) wiederholt werden, wobei die Schritte (a) und (b) gleichzeitig oder ganz oder teilweise sequentiell durchgeführt werden, und
(c) der Prüfung auf die Anwesenheit des verlängerten Primers.

14. Verfahren zur Herstellung einer Polynukleotidsequenz, die mit einer einzelsträngigen Target-Polynukleotidsequenz identisch ist, welches Verfahren umfaßt:
(a) Kombination der einzelsträngigen Target-Polynukleotidsequenz, eines oder mehrerer Enzyme mit DNA-Polymerase- und 3'-Exonuklease-Aktivität, und einer Verlängerungssonde, welche an ihrem 3'-Ende eine Sequenz EP1, die komplementär zu einer Sequenz S1 am 3'-Ende der einzelsträngigen Target-Polynukleotidsequenz ist, und eine Sequenz EP2, die im wesentlichen identisch mit einer Sequenz S2 am 5'-Ende der Target-Sequenz ist, umfaßt, wobei die Verlängerungssonde mindestens ein Phosphorothioat innerhalb der EP2-Sequenz enthält, in einem Medium und
(b) Behandlung des Mediums, um die Hybridisierung der Verlängerungssonde mit dem einzelsträngigen Target-Polynukleotid, die Verlängerung der Verlängerungssonde entlang der einzelsträngigen Target-Polynukleotidsequenz, den Abbau des 3'-Endes der nicht mit der einzelsträngigen Target-Polynukleotidsequenz hybridisierten Verlängerungssonde, um einen Primer zu bilden, der an seinem 3'-Ende die EP2-Sequenz aufweist, die Hybridisierung des Primers mit der verlängerten Verlängerungssonde und die Verlängerung des Primers entlang der verlangerten Verlängerungssonde zu veranlassen.

15. Kit, umfassend in abgepackter Kombination:
eine Verlängerungssonde, die an ihrem 3'-Ende eine Sequenz (EP1) aufweist, die mit einer ersten Sequenz in einer Target-Polynukleotidsequenz hybridisierbar ist, und eine Sequenz (EP2) aufweist, die im wesentlichen identisch mit einer zweiten Sequenz der Target-Polynukleotidsequenz ist, wobei in dieser Target-Polynukleotidsequenz die zweite Sequenz 5' von der ersten Sequenz liegt und nicht daran angrenzt,
eine Nukleotidsequenz (NS), die einen Abschnitt aufweist, der mit EP1 hybridisieren kann, wobei diese NS ein separates Molekül oder Teil der Verlängerungssonde sein kann, und
einen Polydesoxynukleotid-Primer, der mit einer zur zweiten Sequenz komplementären Sequenz hybridisieren kann, wenn kein anderweitiges Mittel zur Bildung des Primers anwesend ist.

16. Kit, umfassend eine Verlängerungssonde mit mindestens einem Phosphorothioat-Diester und eine 3'-Exonuklease, die zur Hydrolysierung der Verlängerungssonde imstande ist.

17. Verfahren nach Anspruch 3 zum Nachweis der Anwesenheit eines Polynukleotid-Analyten, welches Verfahren umfaßt die Schritte:
(a) der Behandlung eines Mediums, von dem angenommen wird, daß es den Polynukleotid-Analyten enthält, um aus dem Polynukleotid-Analyten, falls vorhanden, eine einzelsträngige Target-Polynukleotidsequenz zu bilden, welche Target-Polynukleotidsequenz zwei nicht aneinander angrenzende, nicht-komplementäre Nukleotidsequenzen S1 und S2 aufweist, wobei S2 5' von S1 liegt und mindestens 10 Nukleotide lang ist,
(b) der Kombination des Mediums mit (1) einer Verlängerungssonde, die von zwei Desoxynukleotidsequenzen gebildet wird, wobei die Sequenz am 3'-Ende der Verlängerungssonde (EP1) mit S1 hybridisierbar ist und die andere Desoxynukleotidsequenz (EP2) im wesentlichen identisch mit S2 ist, wobei die Verlängerungssonde mindestens einen Phosphorothioat-Diester enthält und imstande ist, abgebaut zu werden, um einen Polydesoxynukleotid-Primer zu bilden, der mit einer zu S2 komplementären Nukleotidsequenz hybridisieren kann, (3) Desoxynukleosid-Triphosphaten und (4) DNA-Matrizen-abhängiger Polydesoxynukleotid-Polymerase unter Bedingungen, unter denen (A) die Verlängerungssonde mit der Target-Polynukleotidsequenz hybridisiert und daran entlang verlängert wird, um einen Doppelstrang zu bilden, (B) die mit der Target-Nukleotidsequenz nicht hybridisierte Verlängerungssonde abgebaut wird, um den Polydesoxynukleotid-Primer zu bilden, (C) die verlängerte Verlängerungssonde von dem Doppelstrang dissoziiert wird, (D) der Primer mit der verlängerten Verlängerungssonde hybridisiert und daran entlang verlängert wird, (E) der verlängerte Primer von dem Doppelstrang dissoziiert wird, und (F) der Primer mit dem verlängerten Primer hybridisiert und daran entlang verlängert wird, um einen Doppelstrang zu bilden, der verlängerten Primer umfaßt, und die Schritte (E) und (F) wiederholt werden, wobei die Schritte (a) und (b) gleichzeitig oder ganz oder teilweise sequentiell durchgeführt werden, und
(c) der Prüfung auf die Anwesenheit des verlängerten Primers.

18. Verfahren zur Herstellung einer Polynukleotidsequenz, die zu einer einzelsträngigen Target-Polynukleotidsequenz ("Target Sequenz") komplementär ist, welches Verfahren umfaßt die Schritte:
(a) der Hybridisierung des 3'-Endes der Target-Sequenz mit einem Polynukleotid-Primer, welcher einen 3'-Terminus aufweist, der ein Nukleotid-Monophosphat umfaßt, in dem mindestens ein Phosphatsauerstoff durch Schwefel ersetzt ist,
(b) der Verlängerung des Polynukleotid-Primers entlang der Target-Sequenz und
(c) der Dissoziierung des verlängerten Polynukleotid-Primers von der Target-Sequenz.

19. Verfahren nach Anspruch 18, worin 1 bis 5 Phosphatsauerstoffe durch Schwefel ersetzt sind.

20. Verfahren nach Anspruch 18 zur Herstellung einer Polynukleotidsequenz, die eine Sequenz aufweist, welche mit Ausnahme der Anwesenheit einer Phosphor-Schwefel-Bindung anstelle einer Phosphor-Sauerstoff-Bindung mit einer einzelsträngigen Target-Polynukleotidsequenz ("Target-Sequenz") identisch ist, welches Verfahren umfaßt die Schritte:
(a) der Hybridisierung des 3'-Endes der Target-Sequenz mit einem ersten Polynukleotid-Primer ("erster Primer"), der mindestens eine Phosphor-Schwefel-Bindung enthält,
(b) der Verlängerung des ersten Primers entlang der Target-Sequenz,
(c) der Dissoziierung des verlängerten ersten Primers von der Target-Sequenz,
(d) der Hybridisierung des 3'-Endes des verlängerten ersten Primers mit einem zweiten Polynukleotid-Primer ("zweiter Primer"), wobei der zweite Primer gleich dem oder verschieden von dem ersten Primer sein kann, und
(e) der Verlängerung des zweiten Primers entlang des verlängerten ersten Primers, um verlängerten zweiten Primer zu bilden, wobei, wenn die ersten und zweiten Primer gleich sind, der verlängerte erste Primer eine Matrize für den ersten Primer ist, und wenn die ersten und zweiten Primer verschieden sind, der verlängerte erste Primer eine Matrize für den zweiten Primer ist und der verlängerte zweite Primer eine Matrize für den ersten Primer ist

21. Verfahren nach Anspruch 20, worin die ersten und zweiten Primer gleich sind.

22. Verfahren nach Anspruch 20 und 21, worin Bedingungen gewählt werden, um einen wiederholten Zyklus zu erhalten, so daß verlängerte Primer von ihren Matrizen dissoziiert werden und die Primer dann mit den verlängerten Primem hybridisiert und daran entlang verlängert werden.

## Revendications

1. Procédé de production, à partir d'une sonde d'extension et d'une séquence polynucléotidique cible simple brin, d'un polynucléotide dépourvu d'une sonde d'extension non modifiée, ledit polynucléotide ayant une séquence identique a ladite séquence polynucléotidique cible liée au niveau de son extrémité 3' à une séquence polynucléotidique complémentaire d'une séquence polynucléotidique au niveau de l'extrémité 5' de ladite séquence polynucléotidique cible, ledit procédé comprenant :
(a) l'hybridation à l'extrémité 3' de ladite séquence polynucléotidique cible simple brin de l'extrémité 3' de ladite sonde d'extension dans laquelle ladite sonde d'extension contient une séquence essentiellement identique à une séquence S2 à l'extrémité 5' de ladite séquence polynucléotidique cible simple brin,
(b) l'allongement de ladite sonde d'extension le long de ladite séquence polynucléotidique cible simple brin,
(c) la modification de ladite extrémité 3' de ladite sonde d'extension non hybridée à ladite séquence polynucléotidique cible simple brin,
(d) l'hybridation d'une amorce à l'extrémité 3' de la sonde d'extension allongée, ladite amorce comportant ladite séquence S2 à son extrémité 3', et
(e) l'allongement de ladite amorce le long de ladite sonde d'extension allongée.

2. Procédé selon la revendication 1, dans lequel ladite modification comprend un moyen pour allonger ou dégrader ladite extrémité 3' de ladite sonde d'extension non hybridée à ladite séquence polynucléotidique cible simple brin.

3. Procédé de production à partir d'une sonde d'extension d'un polydésoxynucléotide (PDN) ayant deux segments qui sont non juxtaposés et complémentaires l'un de l'autre, ledit PDN étant essentiellement dépourvu de ladite sonde d'extension, ledit procédé comprenant les étapes :
de réunion (a) d'un polynucléotide ayant deux séquences nucléotidiques non juxtaposées, non complémentaires S1 et S2 dans lequel S2 se trouve en 5' de S1 et mesure au moins dix nucléotides de longueur, (b) d'une sonde d'extension constituée de deux séquences désoxynucléotidiques, dans laquelle la séquence à l'extrémité 3' de ladite sonde d'extension, EP1, est susceptible de s'hybrider à S1 et l'autre desdites séquences désoxynucléotidiques, EP2, est homologue à S2, et (c) d'un moyen pour modifier chimiquement l'extrémité 3' de ladite sonde d'extension qui ne s'hybride pas audit polynucléotide, et
d'allongement de ladite sonde d'extension le long dudit polynucléotide dans lequel l'extrémité 3' de ladite sonde d'extension non hybridée audit polynucléotide est modifiée.

4. Procédé selon la revendication 3, dans lequel ledit moyen comprend une séquence nucléotidique EP3 capable de s'hybrider à ladite EP1.

5. Procédé selon la revendication 3, dans lequel ledit moyen comprend une séquence nucléotidique EP3 dans ladite sonde d'extension, ladite EP3 étant capable de s'hybrider à ladite EP1.

6. Procédé selon la revendication 3, dans lequel ledit moyen est un moyen de dégradation de l'extrémité 3' de ladite sonde d'extension.

7. Procédé selon la revendication 6, dans lequel ledit moyen comprend une enzyme douée d'activité 3'-exonucléase.

8. Procédé selon la revendication 6, dans lequel ladite sonde d'extension est dégradée en vue de donner une amorce polydésoxynucléotidique capable de s'hybrider au moins au niveau de son extrémité 3' à une séquence nucléotidique complémentaire de S2.

9. Procédé selon la revendication 3, qui comprend en outre l'inclusion dans ladite combinaison d'une amorce polydésoxynucléotidique capable de s'hybrider au moins au niveau de son extrémité 3' à une séquence nucléotidique complémentaire de S2 dans des conditions telles que (1) ladite sonde d'extension allongée est convertie en une structure simple brin, (2) ladite amorce polydésoxynucléotidique s'hybride à et subit un allongement le long de ladite amorce d'extension allongée en vue de former un duplex comprenant l'amorce allongée, (3) ladite amorce allongée est dissociée dudit duplex, et (4) ladite amorce s'hybride à et subit un allongement le long de ladite amorce allongée de manière à former un duplex comprenant l'amorce allongée.

10. Procédé selon la revendication 9, dans lequel les étapes (3) et (4) sont réitérées.

11. Procédé selon la revendication 3 pour la réplication d'une séquence polynucléotidique cible, ladite séquence polynucléotidique cible ayant deux séquences nucléotidiques non juxtaposées et non complémentaires S1 et S2 mesurant chacune au moine 10 nucléotides de longueur, dans laquelle 52 se trouve en 5' de S1, ledit procédé comprenant l'étape :
de réunion, soit simultanément soit de manière en partie ou totalement séquentielle, (1) de ladite séquence polynucléotidique cible, (2) d'une sonde d'extension ayant deux séquences désoxynucléotidiques dans laquelle la séquence à l'extrémité 3' de ladite sonde d'extension, EP1, est susceptible de s'hybrider à S1 et l'autre desdites séquences désoxynucléotidiques, EP2, est homologue à S2 , (3) d'un moyen pour allonger ou dégrader l'extrémité 3' de ladite sonde d'extension non hybridée à ladite séquence polynucléotidique cible, (4) d'une amorce polydésoxynucléotidique constituée de la séquence S2 à son extrémité 3' où ladite amorce peut être directement introduite ou générée *in situ*, (5) d'ADN-polymérase et (6) de désoxynucléoside-triphosphates dans des conditions telles que (A) une fraction de ladite sonde d'extension s'hybride à et subit un allongement (EP allongée) le long de ladite séquence polynucléotidique cible en formant un duplex, (B) la sonde d'extension non hybridée à ladite séquence nucléotidique cible est allongée ou dégradée à son extrémité 3' (C) ladite EP allongée se dissocie dudit duplex, (D) ladite amorce s'hybride à et subit un allongement le long de ladite EP allongée en formant un duplex comprenant l'amorce allongée, (E) ladite amorce allongée se dissocie dudit duplex, et (F) ladite amorce s'hybride à et subit un allongement le long de ladite amorce allongée en vue de former un duplex comprenant l'amorce allongée, en veillant à répéter les étapes (E) et (F).

12. Procédé selon la revendication 11, dans lequel ladite amorce polydésoxynucléotidique est générée par dégradation de ladite sonde d'extension, et dans lequel le groupe phosphate le plus proche de l'extrémité 3' de ladite amorce est remplacé par un groupe phosphorothioate.

13. Procédé selon la revendication 3 pour la détection de la présence d'une substance polynucléotidique à analyser, ledit procédé comprenant les étapes :
(a) de traitement d'un milieu présumé contenir ladite substance polynucléotidique à analyser en vue de former une séquence polynucléotidique cible simple brin à partir de ladite substance polynucléotidique à analyser, éventuellement présente, ladite séquence polynucléotidique cible ayant deux séquences nucléotidiques non juxtaposées, non complémentaires S1 et S2 dans lesquelles 52 se trouve en 5' de S1, et mesure au moins dix nucléotides de longueur,
(b) de combinaison dudit milieu avec (1) une sonde d'extension ayant deux séquences désoxynucléotidiques dans laquelle la séquence à l'extrémité 3' de ladite sonde d'extension (EP1) est susceptible de s'hybrider à S1 et l'autre desdites séquences désoxynucléotidiques (EP2) est essentiellement identique à S2, (2) une séquence nucléotidique (NS) dont une partie est capable de s'hybrider à EP1 dans laquelle ladite NS peut être une molécule distincte ou une partie de ladite sonde d'extension, (3) une amorce polydésoxynucléotidique capable de s'hybrider à une séquence nucleotidique complémentaire de S2, (4) des désoxynucléoside-triphosphates, (5) et une polydésoxynucléotide-polymérase ADN-dépendante dans des conditions telles que (A) ladite sonde d'extension s'hybride à et subit un allongement (sonde d'extension allongée) le long de ladite séquence polynucléotidique cible en vue de former un duplex, (B) ladite sonde d'extension non hybridée à ladite séquence polynucléotidique cible subit un allongement le long de NS, (C) ladite sonde d'extension allongée se dissocie dudit duplex, (D) ladite amorce s'hybride à et subit un allongement le long de ladite sonde d'extension allongée en vue de former un duplex comprenant l'amorce allongée, (E) ladite amorce allongée se dissocie dudit duplex, et (F) ladite amorce s'hybride à et subit un allongement le long de ladite amorce allongée en vue de former un duplex comprenant l'amorce allongée, en veillant à répéter les étapes (E) et (F), procédé dans lequel (a) et (b) se déroulent simultanément ou de manière en partie ou totalement séquentielle, et
(c) l'analyse de la présence de ladite amorce allongée.

14. Procédé de production d'une séquence polynucléotidique identique à une séquence polynucléotidique cible simple brin, ledit procédé comprenant :
(a) la réunion dans un milieu de ladite séquence polynucléotidique cible simple brin, d'une ou de plusieurs enzymes douées d'activité ADN-polymérase et 3'-exonucléase, et d'une sonde d'extension constituée à son extrémité 3' d'une séquence EPI complémentaire d'une séquence S1 à l'extrémité 3' de ladite séquence polynucléotidique cible simple brin et d'une séquence EP2 qui est essentiellement identique à une séquence S2 à l'extrémité 5' de ladite séquence cible dans laquelle ladite sonde d'extension contient au moins un groupe phosphorothioate au sein de la séquence EP2, et
(b) le traitement dudit milieu en vue d'induire l'hybridation de ladite sonde d'extension audit polynucléotide cible simple brin, l'allongement de ladite sonde d'extension le long de ladite séquence polynucléotidique cible simple brin, la dégradation de l'extrémité 3' de ladite sonde d'extension non hybridée à ladite séquence polynucléotidique cible simple brin en vue de former une amorce ayant à son extrémité 3' ladite séquence EP2, l'hybridation de ladite amorce à la sonde d'extension allongée, et l'allongement de ladite amorce le long de ladite sonde d'extension allongée.

15. Trousse comprenant dans un même coffret :
une sonde d'extension ayant à son extrémité 3' une séquence (EP1) susceptible de s'hybrider à une première séquence dans une séquence polynucléotidique cible et ayant une séquence (EP2) qui est essentiellement identique à une deuxième séquence de ladite séquence polynucléotidique cible, dans laquelle dans ladite séquence polynucléotidique cible ladite deuxième séquence se trouve en 5' et est non juxtaposée à ladite première séquence,
une séquence nucléotidique (NS) dont une partie est capable de s'hybrider à EP1 dans laquelle ladite NS peut être une molécule distincte ou une partie de ladite sonde d'extension, et une amorce polydésoxynucléotidique capable de s'hybrider à une séquence qui est complémentaire de ladite deuxième séquence si le moyen de formation de ladite amorce fait défaut.

16. Trousse comprenant une sonde d'extension comportant au moins un diester de type phosphorothioate et une 3'-exonucléase capable d'hydrolyser ladite sonde d'extension.

17. Procédé selon la revendication 3 pour la détection de la présence d'une substance polynucléotidique à analyser, ledit procédé comprenant les étapes :
(a) de traitement d'un milieu présumé contenir ladite substance polynucléotidique à analyser en vue de former une séquence polynucléotidique cible simple brin à partir de ladite substance polynucléotidique à analyser, éventuellement présente, ladite séquence polynucléotidique cible ayant deux séquences nucléotidiques non juxtaposées, non complémentaires S1 et S2 dans lesquelles S2 se trouve en 5' de S1, et mesure au moins dix nucléotides de longueur,
(b) de combinaison dudit milieu avec (1) une sonde d'extension composée de deux séquences désoxynucléotidiques dans laquelle la séquence à l'extrémité 3' de ladite sonde d'extension (EP1) est susceptible de s'hybrider à S1 et l'autre desdites séquences désoxynucléotidiques (EP2) est essentiellement identique à S2 dans laquelle ladite sonde d'extension contient au moins un diester de type phosphorothioate et est dégradable de manière à former une amorce polydésoxynucléotidique susceptible de s'hybrider à une séquence nucléotidique complémentaire de S2, (3) des désoxynucléoside-triphosphates, et (4) une polydésoxynucléotide-polymérase ADN dépendante dans des conditions telles que (A) ladite sonde d'extension s'hybride à et subit un allongement le long de ladite séquence polynucléotidique cible en vue de former un duplex, (B) ladite sonde d'extension non hybridée à ladite séquence nucléotidique cible est dégradée en vue de former ladite amorce polydésoxynucléotidique, (c) ladite sonde d'extension allongée se dissocie dudit duplex, (D) ladite amorce s'hybride à et subit un allongement le long de ladite sonde d'extension allongée, (E) ladite amorce allongée se dissocie dudit duplex, et (F) ladite amorce s'hybride à et subit un allongement le long de ladite amorce allongée en vue de former un duplex comprenant l'amorce allongée, en veillant à répéter les étapes (E) et (F), procédé dans lequel les étapes (a) et (b) se déroulent simultanément ou de manière en partie ou totalement séquentielle, et
(c) d'analyse de la présence de ladite amorce allongée.

18. Procédé de formation d'une séquence polynucléotidique complémentaire d'une séquence polynucléotidique cible simple brin (" séquence cible "), ledit procédé comprenant les étapes :
(a) d'hybridation à l'extrémité 3' de ladite séquence cible d'une amorce polynucléotidique dont l'extrémité 3' est constituée d'un nucléotide-monophosphate dans lequel un atome de soufre se substitue à au moins un atome d'oxygène du groupe phosphate,
(b) d'allongement de ladite amorce polynucléotidique le long de ladite séquence cible et
(c) de dissociation de ladite amorce polynucléotidique allongée de ladite séquence cible.

19. Procédé selon la revendication 18, dans lequel un atome de soufre se substitue à 1 à 5 atomes d'oxygène du groupe phosphate.

20. Procédé selon la revendication 18 pour former une séquence polynucléotidique ayant une séquence identique, mis à part la présence d'une liaison phosphore-soufre à la place d'une liaison phosphore-oxygène, à une séquence polynucléotidique cible simple brin (" séquence cible "), ledit procédé comprenant les étapes :
(a) d'hybridation à l'extrémité 3' de ladite séquence cible d'une première amorce polynucléotidique (*première amorce ") cpntenant au moins une liaison phosphore-soufre,
(b) d'allongement de ladite première amorce le long de ladite séquence cible,
(c) de dissociation de ladite première amorce allongée de ladite séquence cible,
(d) d'hybridation à l'extrémité 3' de ladite première amorce allongée d'une deuxième amorce polynucléotidique (" deuxième amorce ") dans laquelle ladite deuxième amorce peut être identique ou différente de ladite première amorce, et
(e) d'allongement de ladite deuxième amorce le long de ladite première amorce allongée en vue de former une deuxième amorce allongée dans laquelle, lorsque lesdites première et deuxième amorces sont identiques, ladite première amorce allongée est une matrice pour ladite première amorce et, lorsque lesdites première et deuxième amorces sont différentes, ladite première amorce allongée est une matrice pour ladite deuxième amorce et ladite deuxième amorce allongée est une matrice pour ladite première amorce.

21. Procédé selon la revendication 20, dans lequel lesdites première et deuxième amorces sont identiques.

22. Procédé selon la revendication 20 ou 21, dans lequel les conditions sont choisies de manière à obtenir une série de cycles de sorte que les amorces allongées se dissocient de leurs matrices et lesdites amorces s'hybrident ensuite à et subissent un allongement le long desdites amorces allongées.
